# EUROPEAN PATENT SPECIFICATION

(11) **EP 3 727 203 B1**
(45) Date of publication and mention of the grant of the patent: **22.04.2026**
(21) Application number: 18890927.9
(22) Date of filing: 19.12.2018
(51) Int. Cl.: A61F 2/28, A61F 2/30, A61L 27/44, A61L 27/58, B29C 70/10, A61L 31/12, A61L 31/14, A61B 17/04, A61B 17/80, A61B 17/86, A61B 17/88, A61B 17/00, B29C 70/20, B29L 31/00

(54) **FIBER BUNDLE REINFORCED BIOCOMPOSITE MEDICAL IMPLANTS**
MIT FASERBÜNDEL VERSTÄRKTE MEDIZINISCHE BIOKOMPOSITIMPLANTATE
IMPLANTS MÉDICAUX BIOCOMPOSITES RENFORCÉS PAR FAISCEAUX DE FIBRES

(30) Priority: 20.12.2017 US 201762608542 P
(43) Date of publication of application: 28.10.2020
(73) Proprietor: Ossio Ltd., 3079861 Caesarea (IL)
(72) Inventor: PREISS-BLOOM, Orahn, 3091649 Zichron Yakov (IL); LINDNER, Taly Pnina, 5690500 Savyon (IL); UCHITEL, Ilan Oleg, 4465139 Kfar-Saba (IL); ZEEVI, Tal, 3717227 Pardes Hana-Karkur (IL)
(74) Representative: Birdi, Sandeep Singh
(86) International application number: PCT/IL2018/051377
(87) International publication number: WO 2019/123462

(56) References cited:
- EP-B1- 2 243 500
- US-A- 5 679 299
- US-A1- 2008 255 561
- US-A1- 2009 258 965
- US-A1- 2010 121 463
- US-A1- 2017 246 356
- US-A1- 2017 246 356
- US-B1- 6 171 338
- US-B2- 9 456 890

## Description

### BACKGROUND

### Permanent Orthopedic Implant Materials

Medical implants can be manufactured from metals, alloys, ceramics or both degradable and stable composites. In load-bearing, orthopedic applications that require high strength, usually stainless steel or titanium alloys are used. Metal implants have a long history of successful use in orthopedic surgery but also carry many risks for complications. Although these materials are inert, they are also used in situations in which the need for the implant is only temporary, like in fracture fixation. In the case of metal rods and plates for fracture fixation, a second surgery for device removal may be recommended about one year after confirmation of osseous union. Implant removal causes additional risk and added morbidity for the patient, occupies the availability of clinics, and increases the overall procedure costs. If the device is not removed, it may cause remodeling of the bone. Such remodeling may in turn weaken the bone due to stress shielding or inflammation of the host tissue. The stress shielding can occur due to the high stiffness (modulus) and strength of the metals compared to the stiffness and strength of the cortical bone, so that the metal stresses the bone, which can result in peri prosthetic fractures or loss of bone strength.

Examples of load-bearing medical implants that have traditionally been constructed of metal alloys include bone plates, rods, screws, tacks, nails, clamps, and pins for the fixation of bone fractures and/or osteotomies to immobilize the bone fragments for healing. Other examples include cervical wedges, lumbar cages and plates and screws for vertebral fusion and other operations in spinal surgery.

Biostable polymers and their composites e.g. based on polymethacrylate (PMMA), ultra high molecular weight polyethylene (UHMWPE), polytetrafluoroethylene (PTFE), polyetheretherketone (PEEK), polysiloxane and acrylic polymers have also been used to manufacture medical implants. These materials are not biodegradable or bioresorbable and therefore face many of the same limitations as metals when used for medical implant applications. For example they may require a second surgery for replacing or removing the implant at some point of the lifetime of the implant. Furthermore, these materials are weaker (less strong and stiff) than metal such that they are more susceptible to mechanical failure, particularly after repeated dynamic loading (i.e. through material fatigue or creep).

### Existing degradable polymer medical implants

Resorbable polymers have been used to develop resorbable implants, which can also be referred to as absorbable, bioabsorbable, or biodegradable implants. The advantage of using biocompatible, resorbable polymers is that the polymers, and thus the implant, resorb in the body and release non-toxic degradation products that are cleared by the body. Polymers, including polylactic and polyglycolic acids and polydioxanone, are resorbable biocompatible materials that are currently used as orthopedic plates, rods, anchors, pins or screws for non-load bearing medical implant applications, such as craniofacial applications. These medical implant materials offer the advantage of eventual resorption, eliminating the need for later removal, while allowing stress transfer to the remodeling fracture.

However, current bioabsorbable materials and implants do not have mechanical properties to match metallic implants. The mechanical strength and modulus (approximately 3-5 GPa) of non-reinforced resorbable polymers, is insufficient to support fractured cortical bone, which has an elastic modulus in the range of approximately 15-20 GPa (Snyder SM, et al. measured the bending modulus of human tibial bone to be about 17.5 GPa in Snyder SM Schneider E, Journal of Orthopedic Research, Vol. 9, 1991, pp. 422-431*).* Therefore, the indications of existing medical implants constructed from resorbable polymers are limited and their fixation usually requires protection from motion or significant loading. These devices are only a consideration when fixation of low stress areas is needed (i.e. non-load bearing applications) such as in pediatric patients or in medial malleolar fractures, syndesmotic fixation, maxillofacial, or osteochondral fractures in adults.

### Reinforced degradable polymer materials

Recently, reinforced polymer materials with improved strength and stiffness (modulus) have been introduced. These biodegradable composites comprise polymers reinforced by fillers, usually in fiber form. In composite materials, usually a relatively flexible matrix (i.e. a polymer) is combined with a stiff and strong reinforcement material to enhance the mechanical properties of the composite matrix. For example, biodegradable glass or mineral material can be used to improve the stiffness and strength of a biodegradable polymer matrix. In the background art, several attempts to produce such a composite were reported where bioactive glass particles, hydroxyapatite powder, or short glass fibers were used to enhance the properties of a biodegradable polymer. In most cases, the strength and stiffness of these composites is lower than cortical bone or becomes lower than cortical bone following rapid degradation in a physiological environment. Therefore, the majority of these composite materials are not appropriate for use in load-bearing medical implant applications. However, biodegradable composites with strength and stiffness equivalent to or greater than cortical bone have recently been reported, for example a biodegradable composite comprising a biodegradable polymer and 20-70 vol% glass fibers (WO2010128039 A1). Other composite material implants, for example formed of polymer reinforced with fibers, are disclosed in US Patents 4,750,905, 5,181,930, 5,397,358, 5,009,664, 5,064,439, 4,978,360, 7,419,714.

### Degradation Mechanism of Reinforced Degradable Polymer Materials

When biodegradable composites are used for load-bearing medical implant applications, such as to fixate bone fractures, the mechanical properties of the medical implant must be retained for an extended period. Degradation of the composite will result in premature loss of implant strength or stiffness and can lead to implant function failure, such as insufficient fixation of bone segments resulting in improper bone healing.

Unfortunately, biodegradable composites will begin to hydrolytically degrade once they come into contact with body fluid. This degradation can be a result of degradation of the biodegradable polymer, reinforcing filler, or both. Such degradation in an aqueous environment, such as the physiological environment, can particularly result in a sharp drop-off of mechanical strength and stiffness in certain reinforced polymer materials that are reinforced by inorganic compounds. Where the absorbable polymer matrix is organic material, and the fillers are inorganic compounds, the adhesion between the absorbable polymer matrix and the filler may be reduced by degradation of either the polymer or filler in the aqueous environment and become rapidly reduced such that the initial mechanical properties of the reinforced polymer drop-off rapidly and become less than desirable for adequate load-bearing performance. Aside from the degradation of the polymer and filler separately, poor polymer to reinforcement interface interaction and adhesion can result in early failure at the interface in a aqueous environment, thereby resulting in sharp mechanical property drop off as the reinforcement detaches from the polymer and the reinforcing effect of the filler is lost.

Törmälä et al. (WO 2006/114483) described a composite material containing two reinforcing fibers, one polymeric and one ceramic, in a polymer matrix and reported good initial mechanical results (bending strength of 420 +/-39 MPa and bending modulus of 21.5 GPa) equivalent to the properties of cortical bone. However, the prior art teaches that bioabsorbable composites reinforced with absorbable glass fibers, have a high initial bending modulus but that they rapidly lose their strength and modulus *in vitro.*

While improved interfacial bonding (such as covalent bonding) between the polymer and reinforcement can significantly prolong reinforced bioabsorbable polymer mechanical property retention in an aqueous environment (WO2010128039 A1), continued hydrolysis of the polymer, reinforcement, or interface between the two will result in loss of mechanical properties over time. Since osseous union may take several months or longer, even the prolonged mechanical property degradation profile in covalently bonded reinforced bioabsorbable polymers may be insufficient for optimal function of medical implants used for load-bearing orthopedic applications.

An example of strength loss in a reinforced degradable polymer implant is described with regard to self-reinforced poly-L-lactic acid *(*Majola A et al., Journal of Materials Science Materials in Medicine, Vol. 3, 1992, pp.43-47*).* There, the strength and strength retention of self-reinforced poly-L-lactic acid (SR-PLLA) composite rods were evaluated after intramedullary and subcutaneous implantation in rabbits. The initial bending strength of the SR-PLLA rods was 250-271 MPa. After intramedullary and subcutaneous implantation of 12 weeks the bending strength of the SR-PLLA implants was 100 MPa.

Co- and terpolyesters of PLA, PGA and PCL are of interest in the tailoring of the optimal polymer for resorbable composite material for medical devices. The choice of monomer ratio and molecular weight significantly affects the strength elasticity, modulus, thermal properties, degradation rate and melt viscosity of resorbable composite materials and all of these polymers are known to be degradable in aqueous conditions, both *in vitro* and *in vivo.* Two stages have been identified in the degradation process: First, degradation proceeds by random hydrolytic chain scission of the ester linkages which decreases the molecular weight of the polymers. In the second stage measurable weight loss in addition to chain scission is observed. The mechanical properties are mainly lost or at least a remarkable drop will be seen in them at the point where weight loss starts. Degradation rate of these polymers is different depending on the polymer structure: crystallinity, molecular weight, glass transition temperature, block length, racemization and chain architecture. *(Middleton JC, Tipton AJ,* Biomaterials 21, 2000, 2335-2346*)*

US2017246356 discloses a medical implant comprising a plurality of layers, each layer comprising a polymer and a plurality of uni-directionally aligned continuous reinforcement fibers. US6171338 discloses a surgical device made of biodegradable material for keeping open a tissue cavity, the device including an elongated rod made of the biodegradable material. US9456890(B2) discloses a mammalian tissue scaffold and method for making a tissue scaffold including a rigid scaffold body of biocompatible glass fibers bonded together and in special alignment to define open channels within the scaffold.

### SUMMARY OF THE INVENTION

The background art fails to teach or suggest a reinforced bioabsorbable polymer material exhibiting improved mechanical properties for use in load-bearing medical implant applications, such as structural fixation for load-bearing purposes. The background art fails to teach or suggest such a material where the high strength and stiffness of the implant are retained at a level equivalent to or exceeding cortical bone for a period at least as long as the maximum bone healing time.

The present invention, in at least some embodiments, overcomes these drawbacks of the background art by providing such a reinforced bioabsorbable polymer material, comprising a plurality of fiber bundles for reinforcement. Such fiber bundles enable the material to achieve the high strengths and stiffness required for many medical implant applications. This creates a significant difference from the implant structures, architectures, designs, and production techniques that are known in the art, in which medical implants are produced from polymers or composites comprising individual or layered short or long fiber reinforced polymers. According to the invention, there is provided a medical implant as recited in Claim 1. Certain optional features of the invention are defined in the dependent claims.

Surprisingly, the inventors have found that fiber bundles provide superior strength and other desirable properties, as compared for example to fibers arranged in layers alone, without bundles. With fiber bundle reinforcement, the fibers are preferably aligned such that each fiber or bundle of fibers runs along a path within the composite material. Such alignment means that the bundles provide reinforcement along specific axes within the implant to provide stress resistance where it is most needed. Optionally, the fiber bundles are aligned at up to 70% tolerance, up to 80% tolerance, up to 90% tolerance, up to 95% tolerance or up to 99% tolerance, or any integral number in between.

In regard to tolerance, optionally the fiber bundles may be aligned to twist in a helix formation. The tolerance and/or distance measurements as described herein would also apply to a distance between adjacent bundle segments in the context of the helix.

Preferably, with regard to bioabsorbable fiber bundle - reinforced composite implants, the degradation profile of the composite material within the implant is also taken into consideration, thereby ensuring that the fiber bundles will provide strength and stiffness reinforcement both initially at the initial time of device implantation and also over the course of its functional period within the body.

Mechanical properties that are preferably adjusted for the performance of fiber bundle reinforced implants as described herein include one or more of flexural, tensional, shear, compressional, and torsional strength and stiffness (modulus). For such implants, these properties are preferably meet one or more performance criteria both at time zero (i.e. in the implant following production) and following a period of implantation in the body. The mechanical properties at time zero are dependent on the alignment and orientation of fibers within the part. However, retaining a large percentage of the mechanical properties following implantation in the body (or simulated implantation) requires additional and different considerations.

As will be described in more detail below, such considerations for the medical implant design preferably include one or more of the following parameters: compositions, component ratios, fiber diameters, fiber bundle distribution and alignment, fiber length, etc.

These parameters can impact several additional aspects and properties of the herein described medical implant performance:
1. Material degradation rate (degradation products, local pH and ion levels during degradation)
2. Surface properties that affect interface of implant with surrounding local tissue
3. Biological effects such as anti-microbial or osteoconductive properties
4. Response to sterilization processes (such as ethylene oxide gas, gamma or E-beam radiation)

The present invention provides a solution to these problems by providing, in at least some embodiments, implant compositions from fiber bundle reinforced biocompatible composite materials that are a significant step forward from previous implants in that they can achieve sustainably high, load bearing strengths and stiffness. Furthermore, the biocomposite materials described herein are also optionally and preferably bioabsorbable.

The present invention therefore overcomes the limitations of previous approaches and provides medical implants comprising biodegradable biocomposite compositions featuring fiber bundle reinforcement that have superior mechanical properties and subsequently retain their mechanical strength and stiffness for an extended period.

According to at least some embodiments, there is provided a medical implant, comprising a plurality of reinforcing fiber bundles, each fiber bundle having an axis, comprising a plurality of fibers aligned along the axis of the bundle within 0 to 5 degrees of the axis, and a polymer binding said fiber bundles; wherein said polymer and said fiber bundles are biodegradable; and wherein said fibers are separated by no more than 100 microns within each bundle.

Optionally said fiber bundles are embedded in said polymer. Optionally said fiber bundles are mixed with said polymer. Optionally said alignment of said fibers relative to the fiber bundle axis is between 0 to 1 degree.

Optionally the distance between fibers within the bundle is in the range of 0-50 microns. Optionally the distance between fibers within the bundle is in the range of 0-30 microns. Optionally the distance between fibers within the bundle is in the range of 0-20 microns. Optionally the distance between fibers within the bundle is in the range of 0-10 microns. Optionally the fiber bundles within the medical implant are separated by less than 200 microns. Optionally the fiber bundles within the medical implant are separated by 5-60 microns. Optionally the fiber bundles within the medical implant are separated by 10-40 microns. Optionally the fiber bundles within the medical implant are separated by 10-30 microns. Optionally the fiber bundles within the medical implant are separated by 10-50 microns. Optionally adjacent fiber bundles within the medical implant are offset to each other by an angle of 15 to 75 degrees. Optionally adjacent fiber bundles within the medical implant are offset to each other by an angle of 30 to 60 degrees. Optionally said fibers comprise a reinforcing mineral composition. Optionally mineral content within the implant is in the range of 40%-60% w/w. Optionally mineral content within the implant is in the range of 45%-55% w/w. Optionally mineral content within the bundles is in the range of 40%-70% w/w. Optionally mineral content within the bundles is in the range of 50%-70% w/w.

Optionally the medical implant additionally comprises a compatibilizer wherein weight content of compatibilizer is less than 0.5% w/w. Optionally the polymer comprises L and D isomers of poly lactic acid polymers. Optionally the ratio of L:D isomer of the polymer is in the range of 60:40 to 98:2. Optionally the ratio of L:D isomer of the polymer is in the range of 70:30 to 96:4. Optionally said polymer comprises poly-LD-lactide (PLDLA). Optionally the polymer comprises polylactides (PLA), poly-L-lactide (PLLA), poly-DL-lactide (PDLLA), poly-LD-lactide (PLDLA); polyglycolide (PGA); copolymers of glycolide, glycolide/trimethylene carbonate copolymers (PGA/TMC); other copolymers of PLA, such as lactide/tetramethylglycolide copolymers, lactide/trimethylene carbonate copolymers, lactide/d-valerolactone copolymers, lactide/ε-caprolactone copolymers, L-lactide/DL-lactide copolymers, glycolide/L-lactide copolymers (PGA/PLLA), polylactide-co-glycolide; terpolymers of PLA, such as lactide/glycolide/trimethylene carbonate terpolymers, lactide/glycolide/ ε -caprolactone terpolymers, PLA/polyethylene oxide copolymers; polydepsipeptides; unsymmetrically - 3,6-substituted poly-1 ,4-dioxane-2,5-diones; polyhydroxyalkanoates; such as polyhydroxybutyrates (PHB); PHB/b-hydroxyvalerate copolymers (PHB/PHV); poly-b-hydroxypropionate (PHPA); poly-p-dioxanone (PDS); poly-d-valerolactone - poly-ε-capralactone, poly(ε-caprolactone-DL-lactide) copolymers; methylmethacrylate-N-vinyl pyrrolidone copolymers; polyesteramides; polyesters of oxalic acid; polydihydropyrans; polyalkyl-2-cyanoacrylates; polyurethanes (PU); polyvinylalcohol (PVA); polypeptides; poly-b-malic acid (PMLA): poly-b-alkanbic acids; polycarbonates; polyorthoesters; polyphosphates; poly(ester anhydrides); and mixtures thereof; and natural polymers, such as sugars; starch, cellulose and cellulose derivatives, polysaccharides, collagen, chitosan, fibrin, hyalyronic acid, polypeptides and proteins, or a mixture thereof.

Optionally each fiber bundle comprises between 3-500 reinforcing fibers. Optionally each bundle comprises between 20-300 reinforcing fibers in each bundle. Optionally each bundle comprises between 25-200 reinforcing fibers. Optionally each bundle comprises between 3-100 reinforcing fibers. Optionally each bundle comprises between 5-50 reinforcing fibers. Optionally each bundle comprises between 8-16 reinforcing fibers.

Optionally the diameter of the bundle is from 35 to 6500 microns. Optionally the diameter of the bundle is from 250 to 4000 microns. Optionally the diameter of the bundle is from 325 to 2600 microns. Optionally the diameter of the bundle is from 35 to 1300 microns. Optionally the diameter of the bundle is from 65 to 650 microns.

Optionally the diameter of the bundle is from 100 to 200 microns.

Optionally the fiber bundles are circular in shape. Optionally the fiber bundles are ovular in shape. Optionally said ovular shape comprises a 6:1 ratio of fibers in x-axis to y-axis. Optionally said ratio is 4:1. Optionally said ratio is 3:1. Optionally said ratio is 2:1. Optionally said ratio is 1:1.

Optionally the fiber bundles have a geometry wherein a diameter in any axis of the bundle passing through the center is within 4 times the length of the diameter in any other axis. Optionally said diameter is within 2 times the length. Optionally said diameter is identical. Optionally an average diameter of the fiber bundles is in the range of 0.5mm-10mm. Optionally the average diameter is in the range of 1mm-5mm. Optionally the average diameter is in the range of 1.5mm-3.5mm.

Optionally a fiber density within each fiber bundle is in the range of 30%-99% in terms of average cross-sectional area percentage. Optionally the fiber density is in the range of 40%-95%. Optionally a fiber density within each fiber bundle is in the range of 30%-99% in terms of volume percentage. Optionally said fiber density is in the range of 40%-95%.

Optionally said fibers are longer than 4 mm. Optionally said fibers are longer than 8 mm. Optionally said fibers are longer than 12 mm. Optionally said fibers are longer than 16 mm. Optionally said fibers are longer than 20 mm.

Optionally at least a portion of the reinforcing fibers are of a continuous length at least 50% the longitudinal length of the medical implant. Optionally said length is at least 80% of the length of the medical implant. Optionally said length is at least 100% of the length of the medical implant. Optionally said length is at least 100% of the length of the medical implant and said length is up to 150% of the length of the implant. Optionally said length is at least 100% of the length of the medical implant and is up to 10,000% of the length of the implant. Optionally said length is up to 1000% of the length of the implant. Optionally said length is up to 500% of the length of the implant. Optionally said length is up to 450% of the length of the implant. Optionally said length is up to 400% of the length of the implant. Optionally said length is up to 350% of the length of the implant. Optionally said length is up to 300% of the length of the implant. Optionally said length is up to 250% of the length of the implant. Optionally said length is up to 200% of the length of the implant.

Optionally an average diameter of reinforcing fiber is in the range of 0.1-100 µm. Optionally said diameter is in the range of 1-20 µm. Optionally said diameter is in the range of 8-18 µm.

Optionally a standard deviation of fiber diameter between fibers within the medical implant is less than 5 µm. Optionally said standard deviation of fiber diameter between fibers within the medical implant is less than 3 µm. Optionally said standard deviation of fiber diameter between fibers within the medical implant is less than 1.5 µm.

Optionally a distance between adjacent reinforcing fibers within a biocomposite bundle is in the range of 0-50 µm. Optionally said distance between adjacent fibers is in the range of 1-30 µm. Optionally said distance between adjacent fibers is in the range of 1-20 µm. Optionally said distance between adjacent fibers is in the range of 0-25 µm. Optionally said distance between adjacent fibers is in the range of 0-15 µm. Optionally said distance between adjacent fibers is in the range of 0-10 µm.

Optionally a weight percentage of reinforcing fibers within the biocomposite medical implant is in the range of 20-90%. Optionally said weight percentage is in the range of 40%-70%. Optionally said weight percentage is in the weight range of 40%-60%.

Optionally a volume percentage of reinforcing fibers within the biocomposite medical implant is in the range of 10-80%. Optionally the volume percentage is in the range of 20%-50%.

The term "biodegradable" as used herein also refers to materials that are resorbable, bioresorbable, bioabsorbable or absorbable in the body.

### BRIEF DESCRIPTION OF THE DRAWINGS

Figure 1 depicts the dimensions of the bone plate;
Figure 2 is an SEM of an exemplary bone plate implant; fiber bundles presence and orientation on the plate outer surface are shown;
Figure 3 shows an SEM of an exemplary bone plate implant; fiber bundle on the plate outer surface are shown in close up view;
Figure 4 shows an SEM of an exemplary bone plate implant cross-section; fiber bundle close up depicting fibers diameter range are shown in close up view;
Figure 5 shows an SEM of an exemplary bone plate implant cross-section; showing the distance between fibers within one bundle;
Figure 6 shows an SEM of an exemplary bone plate implant cross-section; showing an example of the distance between bundles;
Figure 7 shows a 3-point flexural bending test apparatus;
Figure 8 shows a suture anchor implant, in a general perspective view;
Figure 9 shows a suture anchor implant in regard to the outer dimensions;
Figures 10A and 10B show micro-CT scans of the suture anchor implant, as a whole (Figure 10A) and in cross-section (Figure 10B);
Figures 11A and 11B depict the test apparatus for the suture anchor pull-out test;
Figure 12 depicts the test apparatus for the suture anchor torsion to failure;
Figure 13 illustrates the single glass fiber geometry as a non-limiting example; and
Figures 14A and 14B show the mode of failure for the two plate types, specifically for the fiber bundle plate (Figure 14A) and the PLDLA plate (Figure 14B).

### DETAILED DESCRIPTION

A medical implant according to at least some embodiments of the present invention is suitable for load-bearing orthopedic implant applications and comprises one or more biocomposite, optionally bioabsorbable, materials where sustained mechanical strength and stiffness are critical for proper implant function. The biocomposite features a plurality of fiber bundles that are aligned along an axis, for reinforcement of the implant.

The present invention, according to at least some embodiments, thus provides fiber bundle reinforced medical implants that are useful as structural fixation for load-bearing purposes, exhibiting sustained mechanical properties as a result of impeded degradation of the bioabsorbable materials that comprise the implant.

Relevant implants may include but are not limited to bone fixation plates, intramedullary nails, joint (hip, knee, elbow) implants, spine implants, and other devices for such applications such as for fracture fixation, tendon reattachment, spinal fixation, and spinal cages.

The reinforcing fibers are preferably continuous fibers. Such continuous fibers are preferably longer than 4 mm, more preferably longer than 8 mm, 12 mm, 16 mm, and most preferably longer than 20 mm.

Alternatively, or in addition, the reinforcing fiber length can be defined as a function of implant length wherein at least a portion of the reinforcing fibers, and preferably a majority of the reinforcing fibers, are of a continuous length at least 50% the longitudinal length of the medical implant or medical implant component that is comprised of these fibers. Preferably, the portion or majority of the reinforcing fibers are of continuous length at least 80% of the length of the medical implant, and more preferably at least 100% of the length of the medical implant. The fibers can be longer than the length of the implant, as can be the length of the fiber bundles. For each, it is possible for the fibers and/or bundles to be at least 100% the length of the implant and up to 150% of the length of the implant, up to 200% of the length of the implant, up to 250% of the length of the implant, up to 300% of the length of the implant, up to 350% of the length of the implant, up to 400% of the length of the implant, up to 450% of the length of the implant, up to 500% of the length of the implant, up to 1000% of the length of the implant, up to 10,000% of the length of the implant, or any percentage in between. Such continuous reinforcing fibers can provide structural reinforcement to a large part of the implant. The average diameter of reinforcing fiber for use with herein reinforced biocomposite medical implant can be in the range of 0.1-100 µm. Preferably, fiber diameter is in the range of 1-20 µm. More preferably, fiber diameter is in the range of 8-16 µm.

The standard deviation of fiber diameter between fibers within the medical implant is preferably less than 5 µm, more preferably less than 3 µm, and most preferably less than 1.5 µm. Uniformity of fiber diameter is beneficial for consistent properties throughout the implant.

Preferably, the weight percentage of reinforcing fibers within the biocomposite medical implant is in the range of 20-90%, more preferably the weight percentage is in the range of 40%-70%, and most preferably in the weight range of 40%-60%.

Preferably, the volume percentage of reinforcing fibers within the biocomposite medical implant is in the range of 10-80%, more preferably the volume percentage is in the range of 20%-50%.

While the biocomposite composition within the implant is important in determining the mechanical and bulk properties of the implant, the specific composition and structure that comes into contact with the surface edge of the implant has unique significance in that this composition and structure can greatly affect how surrounding cells and tissue interact with the implant following implantation into the body. For example, the absorbable polymer part of the biocomposite may be hydrophobic in nature such that it will repel surrounding tissues to a certain degree while the mineral reinforcing fiber part of the biocomposite may be hydrophilic in nature and therefore encourage surrounding tissues to attach to the implant or create tissue ingrowth .

In an optional embodiment of the present invention, the surface presence of one of the compositional components by percentage of surface area is greater than the presence of that component in the bulk composition of the implant by volume percentage. For example, the amount of mineral on the surface might be greater than the amount of polymer, or vice versa. Without wishing to be limited by a single hypothesis, for greater integration with bone, a greater amount of mineral would optionally and preferably be present on the surface. For reduced integration with bone, a greater amount of polymer would optionally and preferably be present on the surface. Preferably, the percentage of surface area composition of one component is more than 10% greater than the percentage of volume percentage of that component in the overall biocomposite implant. More preferably, the percentage is more than 30% greater, and most preferably more than 50% greater.

Optionally, one surface of the medical implant may have a local predominance of one of the biocomposite components while a different surface, or different part of the same surface, may have a local predominance of a different biocomposite component.

Optionally, the medical implant is a threaded screw or other threaded implant. Preferably, the outer layer of the implant will be directionally aligned such that the direction of the fibers approximates the helix angle of the threading. Preferably, the alignment angle of the fiber direction is within 45 degrees of the helix angle. More preferably, the alignment angle is within 30 degrees, and most preferably the alignment angle is within 15 degrees of the helix angle. Approximating the fiber alignment angle to the helix angle in this manner can improve the robustness of the threading and prevent dehiscence of the reinforcing fibers within the threading .

With regard to circular implants, the reinforcing fibers may optionally take the full circular shape of the implant and curve around the circle shape of the implant without deviation from its circumference. Preferably, a portion or a majority of the reinforcing fibers deviate from the circle shape of the implant such that a tangential angle is formed. The tangential angle is defined as the deviation from the direction of the curve at a fixed starting point, where the fixed starting point is the point where the fiber touches or is closest to coming into contact with the center of the cross-sectional circular area.

Preferably the tangential angle between reinforcing fibers within the circular medical implant and the curvature of the implant is less than 90 degrees, more preferably less than 45 degrees.

Preferably the density of the biocomposite composition for use in the present invention is between 1 to 2 g/mL. More preferentially, density is between 1.2 to 1.9 g/mL. Most preferentially between 1.4 to 1.8 g/mL.

### Arranging Fibers in Bundles Provides Additional Strength

Surprisingly, the inventors have found that arranging fibers in bundles provides greater strength for the implant, as opposed to arranging fibers individually or only in layers for example. As used herein, the term "fiber bundle" refers to a bundle of separate fibers, wherein the fibers each run longitudinally in parallel to each other along the length of the bundle. Each fiber is a stand-alone component and preferably comprises a single filament. The fiber bundle contains a number of individual fibers in close proximity to each other but generally with some amount of polymer interspersed between the fibers within the bundle. Preferably, the cross-section of each fiber bundle has an elliptical shape, which may be any type of ellipse, including without limitation a circle or ovoid shape. It is expected that due to this elliptical shape, at least a portion of each fiber bundle cross-section is more approximated, even touching, a neighboring bundle, while at least another portion of each fiber bundle is less approximated to a neighboring bundle.

In the current art of creating components from carbon, filaments made of carbon are typically combined, in the amount of thousands of such filaments, into what is termed a "fiber". The fibers are then used individually to form an object. However this structure is different from the fibers of the present invention, in that each fiber of the present invention is sufficiently thick and strong to be a stand-alone component. The fibers of the present invention are combined into bundles for further advantages, such as strength for example.

For example, US Patent No. 5064439 states that "Preferably, carbon fibers are employed in the present invention. For convenience, the fibers are referred to hereinbelow as carbon fibers ("CF")". Next a method of preparation for such carbon fibers is described as passing bundles of filaments having 3,000-l5,000 filaments/bundle through a solution for coating.

In other words, when the word "fiber" is typically used in regard to carbon fibers in composite compositions, it typically means a fiber which is a bundle of filaments, typically with 3000-15000 filaments per fiber. The individual filaments in the context of carbon fibers are not stand-alone components and there is not generally polymer interspersed between the filaments. The carbon fibers themselves are generally not arranged into fiber bundles.

According to at least some embodiments, there is provided a medical implant, comprising a plurality of reinforcing fiber bundles, each fiber bundle having an axis, comprising a plurality of fibers aligned along the axis of the bundle, and a polymer binding said fiber bundles; wherein said polymer and said fiber bundles are biodegradable; and wherein said fibers are separated by no more than 100 microns within each bundle.

Optionally, the distance between fibers within the bundle is in the range of 0-50 microns. Also optionally, the distance between fibers within the bundle is in the range of 0-30 microns. Preferably, the distance between fibers within the bundle is in the range of 0-20 microns. More preferably, the distance between fibers within the bundle is in the range of 0-10 microns.

According to at least some embodiments, the present invention relates to medical implants comprised of a biocomposite material composition, reinforced by a plurality of fiber bundles. Preferably the biocomposite material composition is comprised of (an optionally bioabsorbable) polymer reinforced by a mineral composition. Preferably the mineral composition reinforcement is provided by a reinforcing fiber made from the mineral composition.

Preferably, the medical implant or part thereof is comprised of a plurality of biocomposite fiber bundles, each bundle comprising a bioabsorbable polymer reinforced by uni-directional reinforcing fibers. The properties of the implant are optionally and preferably at least partially determined according to the fiber bundle composition and dimensions, and the placement of the bundles in regard to the device, for example with regard to fiber bundle direction. The fibers may optionally remain discrete but optionally some melting of the surrounding polymer may occur to bind the bundles together.

A biocomposite fiber bundle can be defined as a continuous or semi-continuous collection of fibers running through part or all of a medical implant, wherein the bundle is comprised of reinforcing fibers that are aligned uni-directionally.

Preferably, there are between 5-2000 reinforcing fibers forming each biocomposite fiber bundle. Preferably, there are between 10- 150 reinforcing fibers in each bundle and most preferably there are between 20-100 reinforcing fibers. Optionally, the reinforcing fibers are continuous. Alternatively, reinforcing fibers may be segmented (i.e. not continuous).

Preferably fiber bundles are roughly circular in shape. Alternatively, fiber bundles are ovular.

Optionally, fiber bundles may take any regular or irregular geometry where the diameter in any axis of the bundle passing through the center is the same or within 4 times the length of the diameter in any other axis, and preferably, within 2 times the length.

Preferably, the average diameter of the fiber bundles is in the range of 0.5mm-10mm. More preferably, the average diameter is in the range of 0.5mm-5mm. Most preferably, the average diameter is in the range of 1 mm-3.5mm.

Preferably the fiber density within each fiber bundle is in the range of 30%-99% in terms of average cross-sectional area percentage, more preferably, in the range of 40%-95%, and most preferably in the range of 45%-70%.

Preferably the fiber density within each fiber bundle is in the range of 30%-99% in terms of weight percentage, more preferably, in the range of 40%-95% and most preferably in the range of 45%-70%.

Optionally, the fiber density within each fiber bundle is at least 3% greater than the overall density of the medical implant. Preferably, at least 5% greater.

Adjacent bundles in this context may mean two distinct adjacent bundles or two adjacent bundle segments where both segments are segments of the same longer fiber bundle.

Optionally, the directional fiber orientation between adjacent fibers within a fiber bundle is the same or similar. Preferably, the average or median angle difference between fibers within the bundle is between 0 to 15 degrees, more preferably between 0 to 10 degrees, and most preferably between 0 to 5 degrees.

The biocomposite fiber bundles within the medical implant are preferably well approximated to each other, within a particular tolerance. Preferably, the average or median distance between adjacent bundles in part or all of the implant, as measured by the distance between the last fiber in one bundle and the first fiber in the subsequent bundle is between 0-200 µm, more preferably between 1-60 µm, 2-40 µm, and most preferably between 3-30 µm. Good approximation of the fibers within a bundle to the fibers within the adjacent bundle allow each bundle to mechanically support the adjacent bundle. However, some distance between the bundles may be desirable to allow for some polymer to remain between the fibers of adjacent bundles and thus adhere them together, to prevent layer dehiscence under high mechanical load.

Adjacent bundles in this context may mean two distinct adjacent bundles or two adjacent bundle segments where both segments are segments of the same longer fiber bundle.

Optionally, the distance between adjacent reinforcing fibers within a biocomposite bundle is in the range of 0-50 µm, preferably the distance between adjacent fibers is in the range of 1-30 µm, more preferably in the range of 1-20 µm, and most preferably in the range of 1-10 µm.

Preferably, the implant preferably comprises between 1-100 biocomposite fiber bundles, more preferably between 1-50 bundles, and most preferably between 3-20 bundles; wherein each bundle may be aligned in a different direction or some of the bundles may be aligned in the same direction as the other bundles.

Preferably, a plurality of fiber bundles are aligned in the direction of the longitudinal axis of the medical implant. Optionally, a majority of fiber bundles are aligned in the direction of the longitudinal axis of the medical implant.

### Bioabsorbable Polymers

In a preferred embodiment of the present invention, the biodegradable composite comprises a bioabsorbable polymer.

The medical implant described herein may be made from any biodegradable polymer. The biodegradable polymer may be a homopolymer or a copolymer, including random copolymer, block copolymer, or graft copolymer. The biodegradable polymer may be a linear polymer, a branched polymer, or a dendrimer. The biodegradable polymers may be of natural or synthetic origin. Examples of suitable biodegradable polymers include, but are not limited to polymers such as those made from lactide, glycolide, caprolactone, valerolactone, carbonates (e.g., trimethylene carbonate, tetramethylene carbonate, and the like), dioxanones (e.g., 1,4-dioxanone), δ-valerolactone, 1,dioxepanones )e.g., 1,4-dioxepan-2-one and 1,5-dioxepan-2-one), ethylene glycol, ethylene oxide, esteramides, γ-ydroxyvalerate, β-hydroxypropionate, alpha-hydroxy acid, hydroxybuterates, poly (ortho esters), hydroxy alkanoates, tyrosine carbonates ,polyimide carbonates, polyimino carbonates such as poly (bisphenol A-iminocarbonate) and poly (hydroquinone-iminocarbonate,(polyurethanes, polyanhydrides, polymer drugs (e.g., polydiflunisol, polyaspirin, and protein therapeutics(and copolymers and combinations thereof. Suitable natural biodegradable polymers include those made from collagen, chitin, chitosan, cellulose, poly (amino acids), polysaccharides, hyaluronic acid, gut, copolymers and derivatives and combinations thereof.

According to the present invention, the biodegradable polymer may be a copolymer or terpolymer, for example: polylactides (PLA), poly-L-lactide (PLLA), poly-DL-lactide (PDLLA), poly-LD-lactide (PLDLA); polyglycolide (PGA); copolymers of glycolide, glycolide/trimethylene carbonate copolymers (PGA/TMC); other copolymers of PLA, such as lactide/tetramethylglycolide copolymers, lactide/trimethylene carbonate copolymers, lactide/d-valerolactone copolymers, lactide/ε-caprolactone copolymers, L-lactide/DL-lactide copolymers, glycolide/L-lactide copolymers (PGA/PLLA), polylactide-co-glycolide; terpolymers of PLA, such as lactide/glycolide/trimethylene carbonate terpolymers, lactide/glycolide/ ε - caprolactone terpolymers, PLA/polyethylene oxide copolymers; polydepsipeptides; unsymmetrically - 3,6-substituted poly-1 ,4-dioxane-2,5-diones; polyhydroxyalkanoates; such as polyhydroxybutyrates (PHB); PHB/b-hydroxyvalerate copolymers (PHB/PHV); poly-b-hydroxypropionate (PHPA); poly-p-dioxanone (PDS); poly-d-valerolactone - poly-ε-capralactone, poly(ε-caprolactone-DL-lactide) copolymers; methylmethacrylate-N-vinyl pyrrolidone copolymers; polyesteramides; polyesters of oxalic acid; polydihydropyrans; polyalkyl-2-cyanoacrylates; polyurethanes (PU); polyvinylalcohol (PVA); polypeptides; poly-b-malic acid (PMLA): poly-b-alkanbic acids; polycarbonates; polyorthoesters; polyphosphates; poly(ester anhydrides); and mixtures thereof; and natural polymers, such as sugars; starch, cellulose and cellulose derivatives, polysaccharides, collagen, chitosan, fibrin, hyalyronic acid, polypeptides and proteins. Mixtures of any of the above-mentioned polymers and their various forms may also be used.

Preferably polymer is PLDLA and ratio of L isomer to D isomer is in the range of 60:40, L:D to 99:1, L:D, and more preferably, the ratio is between 70:30 and 96:4.

### Reinforced Bioabsorbable Polymers

According to at least some embodiments of the present invention, the medical implant comprises a reinforced bioabsorbable polymer (i.e. a bioabsorbable composite that includes the previously described polymer and also incorporates a reinforcing filler, generally in fiber form, to increase the mechanical strength of the polymer).

In a more preferred embodiment of the present invention, the reinforced bioabsorbable polymer is a reinforced polymer composition comprised of any of the above-mentioned bioabsorbable polymers and a reinforcing filler, preferably in fiber form. The reinforcing filler may be comprised of organic or inorganic (that is, natural or synthetic) material. Reinforcing filler may be a biodegradable glass, a cellulosic material, a nano-diamond, or any other filler known in the art to increase the mechanical properties of a bioabsorbable polymer. The filler is preferably made from a material or class of material other than the bioabsorbable polymer itself. However, it may also optionally be a fiber of a bioabsorbable polymer itself.

Numerous examples of such reinforced polymer compositions have previously been documented. For example: A biocompatible and resorbable melt derived glass composition where glass fibers can be embedded in a continuous polymer matrix (EP 2 243 749 A1), Biodegradable composite comprising a biodegradable polymer and 20-70 vol% glass fibers (WO2010128039 A1), Resorbable and biocompatible fiber glass that can be embedded in polymer matrix (US 2012/0040002 A1), Biocompatible composite and its use (US 2012/0040015 A1), Absorbable polymer containing poly[succinimide] as a filler (EP0 671 177 B1).

In a more preferred embodiment of the present invention, the reinforcing filler is bound to the bioabsorbable polymer such that the reinforcing effect is maintained for an extended period. Such an approach has been described in US 2012/0040002 A1 and EP 2243500B1, which discusses a composite material comprising biocompatible glass, a biocompatible matrix polymer and a coupling agent capable of forming covalent bonds.

Preferably, a sizer or compatibilizer is included in the biocomposite implant composition to increase the bond between the polymer and the fiber. Preferably, such compatibilizer or sizer makes up <1% of the overall implant composition by weight and/or by volume. Preferably, such compatibilizer or sizer makes up <0.5% but weight and/or by volume. Most preferably, such compatibilizer or sizer makes up <0.3% by weight and/or by volume.

Preferably, the majority of said compatibilizer or sizer is comprised of a bioabsorbable polymer selected from above-mentioned list of absorbable polymers. Preferably, the polymer within the sizer is of a different composition, intrinsic viscosity, or average molecular weight than the bioabsorbable polymer comprising the polymeric structural component of the implant. Such a compatibilizer is preferably a lower molecular weight (shorter chain) than the polymeric structural component of the implant. Non-limiting examples of such a compatibilizer are given in WO2010122098. For example, optionally the compatibilizer comprises a polymer wherein at least 10% of the structural units of the compatibilizer are identical to the structural units of the structural polymer, and the molecular weight of the compatibilizer is less than 30000 g/mol. Optionally, at least 30 % of the structural units of the compatibilizer are identical to the structural units of the structural polymer and the molecular weight of the compatibilizer is less than 10000 g/mol. More preferably the molecular weight of the compatibilizer is less than 10000 g/mol. Alternatively, 0% of the structural units of the compatibilizer are identical to the structural units of the structural polymer. Within these characteristics, the compatibilizer and the structural polymer are optionally selected independently from the polymeric materials as described herein. As noted above, the biodegradable composite and fibers are preferably arranged in the form of biodegradable composite fiber bundles, where each bundle comprises uni-directionally aligned continuous reinforcement fibers embedded in a polymer matrix comprised of one or more bioabsorbable polymers.

The biodegradable composite is preferably embodied in a polymer matrix, which may optionally comprise any of the above polymers. Optionally and preferably, it may comprise a polymer selected from the group consisting of PLLA (poly-L-lactide), PDLLA (poly-DL-lactide), PLDLA, PGA (poly-glycolic acid), PLGA (polylactide-glycolic acid), PCL (Polycaprolactone), PLLA-PCL and a combination thereof. If PLLA is used, the matrix preferably comprises at least 30% PLLA, more preferably 50%, and most preferably at least 70% PLLA. If PDLA is used, the matrix preferably comprises at least 5% PDLA, more preferably at least 10%, most preferably at least 20% PDLA.

Preferably, the inherent viscosity (IV) of the polymer matrix (independent of the reinforcement fiber) is in the range of 1.2 to 2.4 dl/g, more preferably in the range of 1.5 to 2.1 dl/g.

Inherent Viscosity (IV) is a viscometric method for measuring molecular size. IV is based on the flow time of a polymer solution through a narrow capillary relative to the flow time of the pure solvent through the capillary.

Preferably, the average molecular weight of the polymer matrix, as measured by GPC, is in the range of 100 kDa - 400 kDa. More preferably, the average molecular weight is in the range of 120 kDa - 250 kDa. Most preferably, the average molecular weight is in the range of 150 kDa - 250 kDa.

### Reinforcement Fiber

Preferably, reinforcement fiber is comprised of silica-based mineral compound such that reinforcement fiber comprises a bioresorbable glass fiber, which can also be termed a bioglass fiber composite.

Bioresorbable mineral fiber may optionally have oxide compositions in the following mol.% ranges:
Na₂O: 10.0 - 19.0 mol.%
CaO: 9.0 - 14.0 mol.%
MgO: 1.5 - 8.0 mol.%
B₂O₃: 0.5 - 3.0 mol.%
Al₂O₃: 0 - 0.8 mol.%
P₂O₃: 0.1 - 0.8 mol.%
SiO₂: 67 - 73 mol.%
K₂O: 0 - 0.8 mol.%

And more preferably in the following mol.% ranges:
Na₂O: 11.5 - 13.0 mol.%
CaO: 9.0 - 10.0 mol.%
MgO: 7.0 - 8.0 mol.%
B₂O₃: 1.4 - 2.0 mol.%
P₂O₃: 0.5 - 0.8 mol.%
SiO₂: 67 - 70 mol.%
K₂O: 0 - 0.4 mol.%

Alternatively, above mineral composition ranges are applicable as weight % (w/w) rather than as mol%.

Additional optional glass fiber compositions have been described previously by Lehtonen TJ et al. (Acta Biomaterialia 9 (2013) 4868-4877), which is included here by reference in its entirety; such glass fiber compositions may optionally be used in place of or in addition to the above compositions.

Additional optional bioresorbable glass compositions are described in the following patent applications: Biocompatible composite and its use (WO2010122098); and Resorbable and biocompatible fibre glass compositions and their uses (WO2010122019).

### Optional Additional Features

The below features and embodiments may optionally be combined with any of the above features and embodiments.

Tensile strength of the reinforcement fiber is preferably in the range of 1200-2800 MPa, more preferably in the range of 1600-2400 MPa, and most preferably in the range of 1800-2200 MPa.

Elastic modulus of the reinforcement fiber is preferably in the range of 30-100 GPa, more preferably in the range of 50-80 GPa, and most preferably in the range of 60-70 GPa.

Optionally, a majority of reinforcement fibers aligned to the longitudinal axis of the medical implant are of a length of at least 50% of the total length of the implant, preferably at least 60%, more preferably at least 75%, and most preferably at least 85%.

### Medical Implant Composite Structure

Implant may be selected from a group that includes orthopedic pins, screws, plates, intramedullary rods, hip replacement, knee replacement, meshes, etc.

The average wall thickness in the implant is preferably in the range of 0.2 to 10 mm, more preferably in the range of 0.4 to 5 mm, more preferably in the range of 0.5 to 2 mm, and most preferably in the range of 0.5 to 1.5 mm.

Optionally, implant may comprise reinforcing ribs, gussets, or struts.

Rib base thickness is preferably more than 20% of adjoining wall thickness, more preferably more than 30%, and most preferably more than 50% of adjoining wall thickness.

Preferably, rib height is at least 2.0 times the adjoining wall thickness, more preferably at least 3.0 times the wall thickness.

Draft angle of reinforcing ribs is preferably between 0.2-3.0°, more preferably between 0.4-1.0°.

Preferably, distance between the center of the ribs is at least 2 times adjoining wall thickness. More preferably, at least 3 times adjoining wall thickness.

Optionally, the ribs can be diagonal and conjoined at the end.

Optionally, ribs along one axis, for example the longitudinal axis of the implant, are taller than the ribs along the perpendicular axis, for example the latitudinal axis of the implant, in order to facilitate easier insertion of the implant.

Optionally, the implant may comprise one or more bosses to accommodate screw insertion. Preferably, the boss is between 2-3 times the screw diameter for self-tapping screw applications. Boss may additionally include supportive gusses or ribs.

Optionally, one or more sides of implant may be textured.

Optionally, implant may contain continuous fibers aligned in a circular arrangement around holes, such as screw or pin holes, within the implant.

### Perforated implant part walls

In some medical implants, it is desirable for there to be cellular or tissue ingrowth through the implant so as to strengthen the incorporation of the implant into the tissue and to increase compliance of the implant in physiological function. In order to further promote such ingrowth, it is beneficial to have gaps or holes in the walls of the herein described medical implant.

Preferably, if present, such perforations in implant walls comprise at least 10% of the surface area of the implant, more preferably at least 20%, at least 30%, at least 40%, or at least 50% of the surface area of the implant.

In one optional embodiment of the present invention, the implant is a screw and the fenestrations of the threading contain perforation.

In a preferred embodiment, a majority of perforations are between reinforcement fibers and do not penetrate reinforcement fibers.

### Cages of bone filler

In another embodiment of the present invention, the implant comprises an orthopedic implant and the implant forms a partial or full container and an osteoconductive or osteoinductive material is contained within the implant container.

In a preferred embodiment, the implant container is additionally perforated so as to allow improved bone ingrowth into the osteoconductive or osteoinductive material contained within the implant cage.

In an optional embodiment, the implant comprises an opening or door through which bone filler can be introduced and/or bone ingrowth can take place.

In an optional embodiment, the implant comprises two or more discrete parts or separate parts joined by a joint such that implant cage may be filled with bone filler material and subsequently assembled or closed to trap bone filler inside.

### Framework of continuous fiber reinforced structure with non-reinforced surrounding material

In one embodiment of the present invention, medical implant comprises a structural support comprised of a continuous fiber-reinforced bioabsorbable composite material and additionally comprises a section comprised of non-reinforced polymer material.

Optionally the non-reinforced polymer section is a bone interface layer and dimensions of the interface layer are partially or entirely determined by the bone geometry of a specific patient or patient population.

Optionally the bone geometry of patient or patient population is determined by measuring through imaging technique such as X-Ray, CT, MRI.

Optionally the elastic modulus and/or flexural strength of structural support is at least 20% greater than that of the non-reinforced polymer section.

Optionally, continuous-fiber reinforced composite material in implant is coated with a polymer resin wherein the polymer resin on fiber in the composite material has a higher or lower melting temp than the flowable matrix resin; or polymer resin on fiber has slower or faster degradation rate than flowable matrix resin; or polymer resin on fiber is more hydrophobic or more hydrophilic than flowable matrix resin

In an optional embodiment, an additional section or layer is comprised of a reinforced polymer but where polymer is reinforced by non-continuous fibers, preferably fibers less than 10mm in length, and more preferably less than 5mm in length.

In an optional embodiment, an additional section or layer of non-reinforced or non-continuous fiber reinforced polymer additional comprises an additive.

Optionally, additive comprises an osteoconductive material or combination of osteoconductive materials such as beta tricalcium phosphate, calcium phosphate, hydroxyapatite, decellularized bone.

Optionally, the additive comprises an anti-microbial agent or bone inducing agent.

### Production Method

Continuous-fiber reinforced bioabsorbable implants may optionally be produced using any method known in the art. Preferably, implant is primarily produced by method other than injection molding. More preferably, implant is primarily produced using manufacturing method that subjects implant to compressive pressure, such as compression molding.

Preferably, moisture content of implant following molding is less than 30%, more preferably less than 20%, even more preferably less than 10%, 8%, 6%, 5%.

### Fabrication of the Implant

Any of the above-described bioabsorbable polymers or reinforced bioabsorbable polymers may be fabricated into any desired physical form for use with the present invention. The polymeric substrate may be fabricated for example, by compression molding, casting, injection molding, pultrusion, extrusion, filament winding, composite flow molding (CFM), machining, or any other fabrication technique known to those skilled in the art. The polymer may be made into any shape, such as, for example, a plate, screw, nail, fiber, sheet, rod, staple ,clip, needle, tube, foam, or any other configuration suitable for a medical device.

### Load-bearing mechanical strength

The present invention particularly relates to bioabsorbable composite materials that can be used in medical applications that require high strength and a stiffness compared to the stiffness of bone. These medical applications require the medical implant to bear all or part of the load applied by or to the body and can therefore be referred to generally as "load-bearing" applications. These include fracture fixation, tendon reattachment, joint replacement, spinal fixation, and spinal cages.

The flexural strength preferred from the herein described load-bearing medical implant is at least 200 MPa, preferably above 400 MPa, more preferably above 600 MPa, and even more preferably above 800 MPa. The Elastic Modulus (or Young's Modulus) of the bioabsorbable composite for use with the present invention is preferably at least 5 GPa, more preferably above 10 GPa, and even more preferably above 15 GPa, 20 GPa but not exceeding 100 GPa and preferably not exceeding 60 GPa.

### Sustained mechanical strength

There is a need for the bioabsorbable load-bearing medical implants of the present invention to maintain their mechanical properties (high strength and stiffness) for an extended period to allow for sufficient bone healing. The strength and stiffness preferably remains above the strength and stiffness of cortical bone, approximately 150-250 MPa and 15-25 GPa respectively, for a period of at least 3 months, preferably at least 6 months, and even more preferably for at least 9 months *in vivo* (i.e. in a physiological environment).

More preferably, the flexural strength remains above 400 MPa and even more preferably remains above 600 MPa.

In another embodiment of the present invention, the mechanical strength degradation rate of the coated medical implant approximates the material degradation rate of the implant, as measured by weight loss of the biodegradable composite.

In a preferred embodiment, the implant retains greater than 50% of its mechanical strength after 3 months of implantation while greater than 50% of material degradation and hence weight loss occurs within 12 months of implantation.

In a preferred embodiment, the implant retains greater than 70% of its mechanical strength after 3 months of implantation while greater than 70% of material degradation and hence weight loss occurs within 12 months of implantation.

In a preferred embodiment, the implant retains greater than 50% of its mechanical strength after 6 months of implantation while greater than 50% of material degradation and hence weight loss occurs within 9 months of implantation.

In a preferred embodiment, the implant retains greater than 70% of its mechanical strength after 6 months of implantation while greater than 70% of material degradation and hence weight loss occurs within 9 months of implantation.

The mechanical strength degradation and material degradation (weight loss) rates of the medical implant can be measured after *in vivo* implantation or after *in vitro* simulated implantation. In the case of *in vitro* simulated implantation, the simulation may be performed in real time or according to accelerated degradation standards.

"Biodegradable" as used herein is a generalized term that includes materials, for example polymers, which break down due to degradation with dispersion *in vivo.* The decrease in mass of the biodegradable material within the body may be the result of a passive process, which is catalyzed by the physicochemical conditions (e.g. humidity, pH value) within the host tissue. In a preferred embodiment of biodegradable, the decrease in mass of the biodegradable material within the body may also be eliminated through natural pathways either because of simple filtration of degradation by-products or after the material's metabolism ("Bioresorption" or "Bioabsorption"). In either case, the decrease in mass may result in a partial or total elimination of the initial foreign material. Elimination of the initial foreign material may include complete dispersion *in vivo* or may additionally include incorporation or remodeling of part of the initial foreign material into the surrounding *in vivo* environment. In a preferred embodiment, said biodegradable composite comprises a biodegradable polymer that undergoes a chain cleavage due to macromolecular degradation in an aqueous environment.

A polymer is "absorbable" within the meaning of this invention if it is capable of breaking down into small, non-toxic segments which can be metabolized or eliminated from the body without harm. Generally, absorbable polymers swell, hydrolyze, and degrade upon exposure to bodily tissue, resulting in a significant weight loss. The hydrolysis reaction may be enzymatically catalyzed in some cases. Complete bioabsorption, i.e. complete weight loss, may take some time, although preferably complete bioabsorption occurs within 24 months, most preferably within 12 months.

The term "polymer degradation" means a decrease in the molecular weight of the respective polymer. With respect to the polymers, which are preferably used within the scope of the present invention said degradation is induced by free water due to the cleavage of ester bonds. The degradation of the polymers as for example used in the biomaterial as described in the examples follows the principle of bulk erosion. Thereby a continuous decrease in molecular weight precedes a highly pronounced mass loss. Said mass loss is attributed to the solubility of the degradation products. Methods for determination of water induced polymer degradation are well known in the art such as titration of the degradation products, viscometry, differential scanning calorimetry (DSC).

The term "Biocomposite" as used herein is a composite material formed by a matrix and a reinforcement of fibers wherein both the matrix and fibers are biocompatible and optionally bioabsorbable. In most cases, the matrix is a polymer resin, and more specifically a synthetic bioabsorbable polymer. The fibers are optionally and preferably of a different class of material (i.e. not a synthetic bioabsorbable polymer), and may optionally comprise mineral, ceramic, cellulosic, or other type of material.

### Clinical Applications

The medical implants discussed herein are generally used for bone fracture reduction and fixation to restore anatomical relationships. Such fixation optionally and preferably includes one or more, and more preferably all, of stable fixation, preservation of blood supply to the bone and surrounding soft tissue, and early, active mobilization of the part and patient.

There are several exemplary, illustrative, non-limiting types of bone fixation implants for which the materials and concepts described according to at least some embodiments of the present invention may be relevant, as follows:

### Bone Plate

A bone plate is typically used to maintain different parts of a fractured or otherwise severed bone substantially stationary relative to each other during and/or after the healing process in which the bone mends together. Bones of the limbs include a shaft with a head at either end thereof. The shaft of the bone is generally elongated and of relatively cylindrical shape.

It is known to provide a bone plate which attaches to the shaft or head and shaft of a fractured bone to maintain two or more pieces of the bone in a substantially stationary position relative to the one another. Such a bone plate generally comprises a shape having opposing substantially parallel sides and a plurality of bores extending between the opposing sides, wherein the bores are suitable for the receipt of pins or screws to attach the plate to the bone fragments.

For proper function of the bone plate in maintaining different parts of a fractured bone stationary relative to each other, the plate must be of sufficient mechanical strength and stiffness to maintain the position of the bone fragments or pieces. However, it must achieve these mechanical properties within a low profile thickness profile to ensure that there will be sufficient space for the bone plate to fit between bone and the surrounding soft tissue. The thickness of the bone plate is generally in the range of 2.0 mm to 8.0 mm and more commonly in the range of 2.0 mm to 4.0 mm. The widths of the plates are variable but

### Screws

Screws are used for internal bone fixation and there are different designs based on the type of fracture and how the screw will be used. Screws come in different sizes for use with bones of different sizes. Screws can be used alone to hold a fracture, as well as with plates, rods, or nails. After the bone heals, screws may be either left in place or removed.

Screws are threaded, though threading can be either complete or partial. Screws can include compression screws, locking screws, and/or cannulated screws. External screw diameter can be as small as 0.5 or 1.0 mm but is generally less than 3.0mm for smaller bone fixation. Larger bone cortical screws can be up to 5.0mm and cancellous screws can even reach 7-8 mm. Some screws are self-tapping and others require drilling prior to insertion of the screw. For cannulated screws, a hollow section in the middle is generally larger than 1mm diameter in order to accommodate guide wires.

### Wires/Pins

Wires are often used to pin bones back together. They are often used to hold together pieces of bone that are too small to be fixed with screws. They can be used in conjunction with other forms of internal fixation, but they can be used alone to treat fractures of small bones, such as those found in the hand or foot. Wires or pins may have sharp points on either one side or both sides for insertion or drilling into the bone.

"K-wire" is a particular type of wire generally made from stainless steel, titanium, or nitinol and of dimensions in the range of 0.5 - 2.0 mm diameter and 2-25 cm length. "Steinman pins" are general in the range of 2.0 - 5.0 mm diameter and 2-25 cm length. Nonetheless, the terms pin and wire for bone fixation are used herein interchangeably.

### Anchors

Anchors and particularly suture anchors are fixation devices for fixing tendons and ligaments to bone. They are comprised of an anchor mechanism, which is inserted into the bone, and one or more eyelets, holes or loops in the anchor through which the suture passes. This links the anchor to the suture. The anchor which is inserted into the bone may be a screw mechanism or an interference mechanism. Anchors are generally in the range of 1.0 - 6.5 mm diameter

### Cable, ties, wire ties

Cables, ties, or wire ties can be used to perform fixation by cerclage, or binding, bones together. Such implants may optionally hold together bone that cannot be fixated using penetration screws or wires/pin, either due to bone damage or presence of implant shaft within bone. Generally, diameter of such cable or tie implants is optionally in the range of 1.0 mm - 2.0 mm and preferably in the range of 1.25 - 1.75 mm. Wire tie width may optionally be in the range of 1 - 10 mm.

### Nails or Rods

In some fractures of the long bones, medical best practice to hold the bone pieces together is through insertion of a rod or nail through the hollow center of the bone that normally contains some marrow. Screws at each end of the rod are used to keep the fracture from shortening or rotating, and also hold the rod in place until the fracture has healed. Rods and screws may be left in the bone after healing is complete. Nails or rods for bone fixation are generally 20-50 cm in length and 5-20 mm in diameter (preferably 9-16mm). A hollow section in the middle of nail or rod is generally larger than 1mm diameter in order to accommodate guide wires.

Any of the above-described bone fixation implants may optionally be used to fixate various fracture types including but not limited to comminuted fractures, segmental fractures, non-union fractures, fractures with bone loss, proximal and distal fractures, diaphyseal fractures, osteotomy sites, etc.

### Example #1 - Biocomposite Medical Implant Bone Plate

The example below describes an orthopedic fixation plate implant produced from reinforced biocomposite material containing reinforcing fiber bundles.

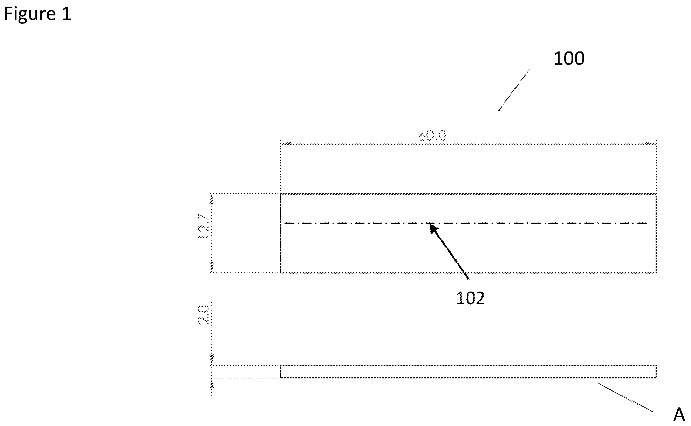

For proper function of the bone plate in maintaining different parts of a fractured bone stationary relative to each other, the plate must be of sufficient mechanical strength and stiffness to maintain the position of the bone fragments or pieces. The bone plate thickness in this example is 2mm thick (shown in the "A" view of Figure 1) and has straight fiber bundles aligned with the plate's longitudinal axis in order to increase the plate resistance to flexural bending in the most critical direction. The fibers within the fiber bundle are aligned along the axis of the fiber bundle (approximately 0 degrees angle to the axis) and are bound together with bioabsorbable polymer.

Figure 1 depicts the dimensions of the bone plate. Figure 1 shows a bone plate 100 with a fiber bundle alignment axis 102, shown also in a side view ("A"). The top view shows exemplary dimensions of 60 mm long and 12.7 mm wide, as well as the thickness of 2 mm, all of which could be changed for various applications.

### Materials and Preparations

Material composite was comprised of PLDLA 70/30 polymer reinforced with 50% w/w, 70%, or 85% w/w continuous mineral fibers. The mineral fibers' composition was approximately Na₂O 14%, MgO 5.4%, CaO 9%, B₂O₃ 2.3%, P₂O₅ 1.5%, and SiO₂ 67.8% w/w. The bone plate testing samples were manufactured by compression molding fiber bundle reinforced biocomposite material into a designated single cavity mold. Biocomposite material comprised the PLDLA polymer with embedded uni-directionally aligned continuous fiber bundles. Orientation of fibers to each fiber bundle was approximately 0°. Orientation of layers relative to longitudinal axis of plate were approximately 0°.

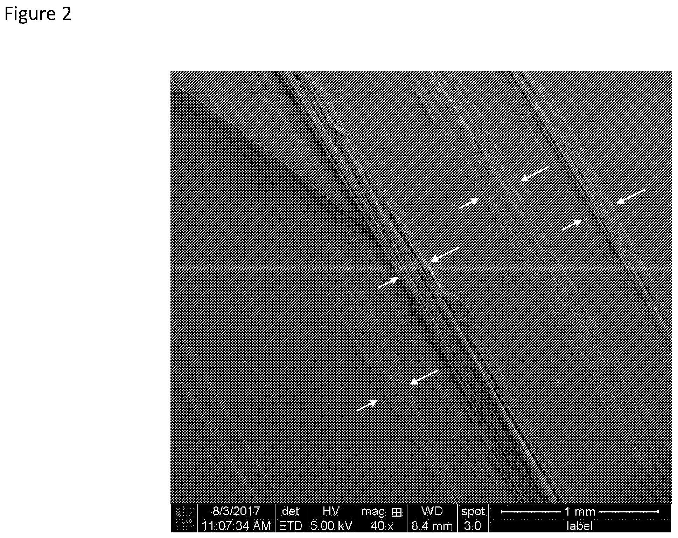

The resultant plate has straight fiber bundles along the longitudinal axis of plate. Fiber bundles presence and orientation is apparent on the outer surface of the bone plate (Figures 2, 3 and 4).

Figure 2 is an SEM of an exemplary bone plate implant; the presence and orientation of fiber bundles on the plate outer surface are shown. A top down view of the surface of implant plate is shown, where the width of some fiber bundles can be observed exposed through the polymer surface of the implant. The width (i.e. diameter) of each bundle is between 5-10 fibers wide such that there are between 20-80 fibers in each bundle depicted herein. The width is indicated with arrows.

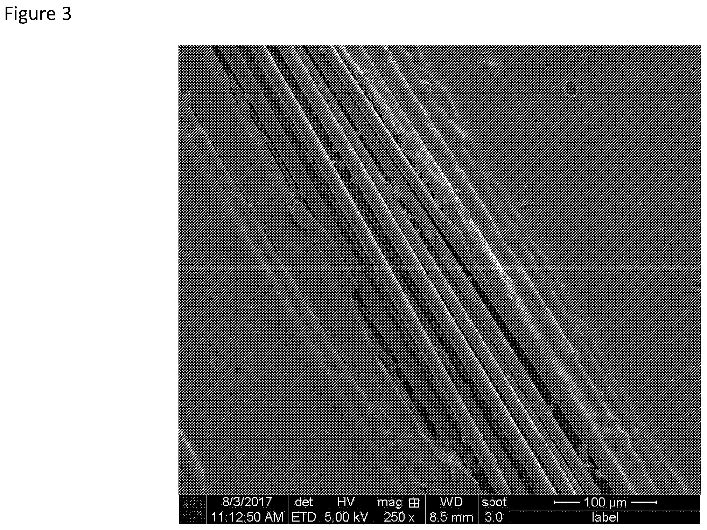

Figure 3 shows an SEM of an exemplary bone plate implant; fiber bundles on the plate outer surface are shown in close up view. Individual fibers are clearly visible in Figure 3. The fiber diameter within each bundle ranges from 9.5µm to 16.8 µm. A top down view of the surface of the implant plate is shown, where the width of one fiber bundle can be observed exposed through the polymer surface of the implant. The width (i.e. diameter) of this bundle is approximately (only partially visible) 10 fibers wide such that there are about 80 fibers in this bundle.

As can be seen in Figures 2 and 3, relatively few fibers are present in each bundle, in comparison to technologies such as carbon fiber filaments, in which each such bundle would typically feature thousands of such filaments.

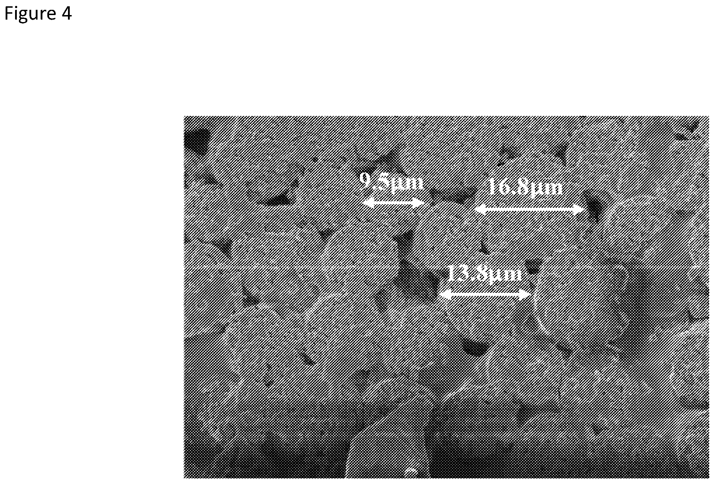

Figure 4 shows a representative measurement of fibers within one bundle at a cross-section of the bone plate. Figure 4 shows an SEM of an exemplary bone plate implant cross-section; fiber bundle close up depicting fibers diameter range are shown in close up view. In particular, the SEM is of a close up view of fibers within a fiber bundle from cross-section cut of the fiber bundle. The fiber width (diameter) of various fibers in the cross-section is shown as 9.5 microns, 13.8 microns and 16.8 microns (arrows indicate width).

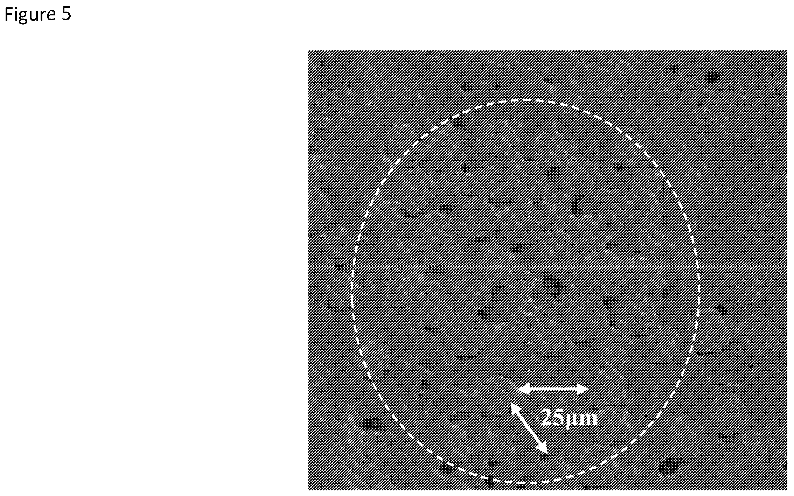

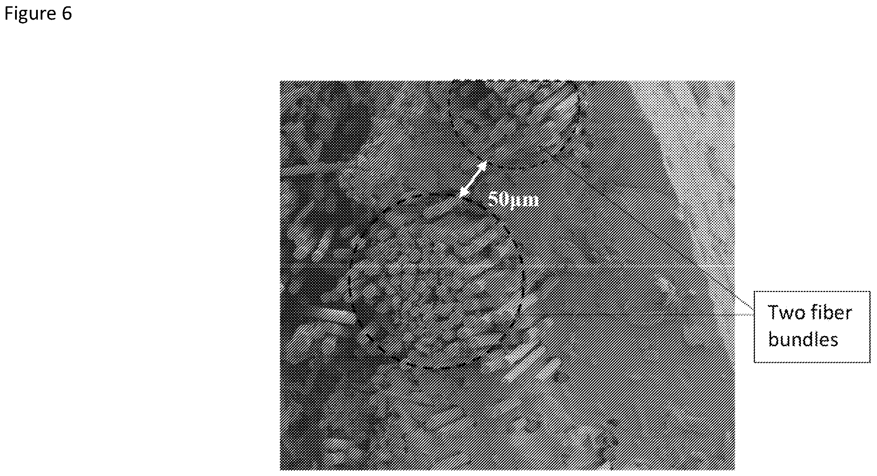

Figure 5 shows an SEM of an exemplary bone plate implant cross-section; showing the distance between fibers within one bundle. The SEM is of a view of a fiber bundle (boundaries indicated by a dotted line) from a cross-sectional cut of the fiber bundle. Distances between fibers within bundle vary from complete approximation (0 um) up to 25 microns between such fibers. Figure 6 shows an SEM of an exemplary bone plate implant cross-section; showing an example of the distance between bundles. The boundaries of two fiber bundles are indicated with dotted lines. The number of fibers within fiber bundles ranges from 50 fibers (Figure 5) to approximately 150 fibers per bundle (Figure 6).

The distance between fibers within the bundle ranges from 0 to 15 µm in some of the bundles and from 0 to 25 µm in other bundles. Figure 5 shows an example of such distances. The distance between one fiber bundle to another ranges from 0 to 50 µm (Figure 6).

### EXAMPLE 2 - Additional bone plate testing

### Methods

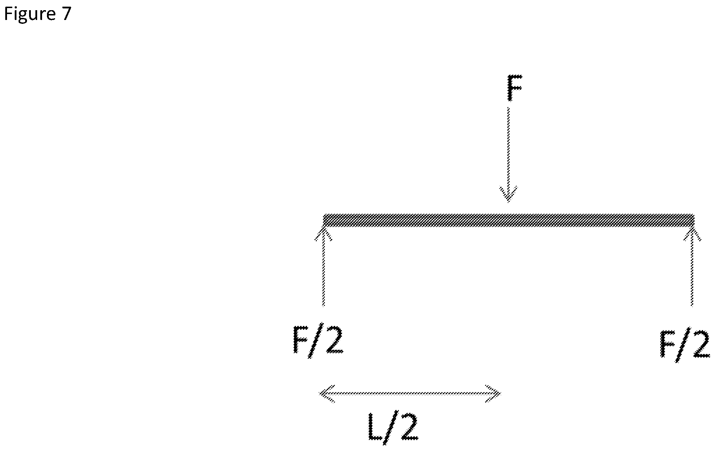

Two types of bone plate implants were tested for flexural bending for comparison. One plate is the fiber bundle plate described above, with longitudinal unidirectional fiber bundles. The second plate is a multidirectional layers plate, which was produced with alternating unidirectional biocomposite fiber-reinforced material layers orientations of 0° and 45° to the plate axis. Each layer was approximately 0.18 mm in thickness. The samples were tested for flexural bending using a 3-point bending method in accordance with ASTM D790. Figure 7 shows a 3-point flexural bending test apparatus.

The load was applied to the middle of the specimen at a deflection rate of 2 mm/min, with the supports 32 mm apart. Load and displacement were measured and recorded by the TestResources Single Column Test Machine, model 220 Frame-1505017-10F with a 5kN load cell (S/N 040017). The maximum load reached was recorded as the Maximum flexural load.

### Results

Table 1 details the averaged values of maximum load, the calculated flexural strength and Young modulus and their standard deviations.

**Table 1 - Mean values and standard deviations of the mechanical properties and bulk properties of the implants (n=3).**

| Sample type | Max Load [N] | Flexural strength [MPa] |
|---|---|---|
| Fiber bundle plate | 542.33 ± 93.24 | 1024.88 ± 176.21 |
| Fiber layers plate | 313.67 ± 63.67 | 592.76 ± 120.89 |

The bone plate implant produced with unidirectional fiber bundles results were significantly higher than for the layers plate. Mean maximal load at failure and the mean flexural strength were ~73% higher for the fiber bundle plate relative to the layers plate. These values reflect a distinct mechanical superiority of the fiber bundle plate over the layers plate in the most critical mode of failure expected for the bone plate implant.

### Example #3 - Biocomposite Medical Implant Suture Anchor

The example below describes a suture anchor implant produced from reinforced biocomposite material containing reinforcing fiber bundles.

Suture anchors are fixation devices for fixing tendons and ligaments to bone. They are comprised of an anchor mechanism, which is inserted into the bone, and one or more eyelets, holes or loops in the anchor through which the suture passes. This links the anchor to the suture. The anchor which is inserted into the bone may be a screw mechanism or an interference mechanism. An interference mechanism also involves a screw, but involves place the suture next to the screw and making a hole in the corresponding bodily tissue, such as bone for example.

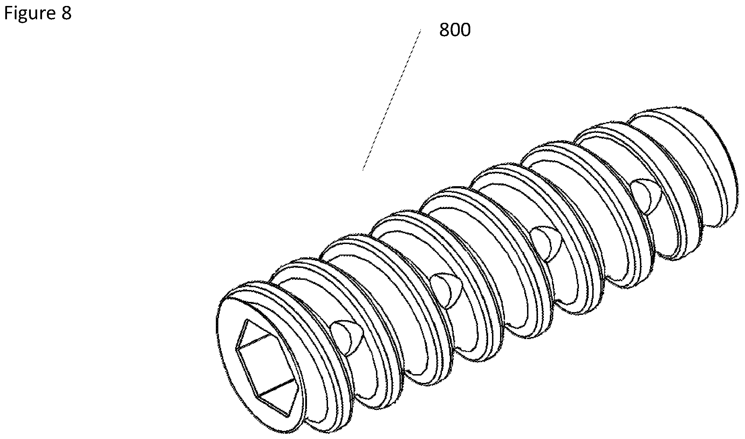

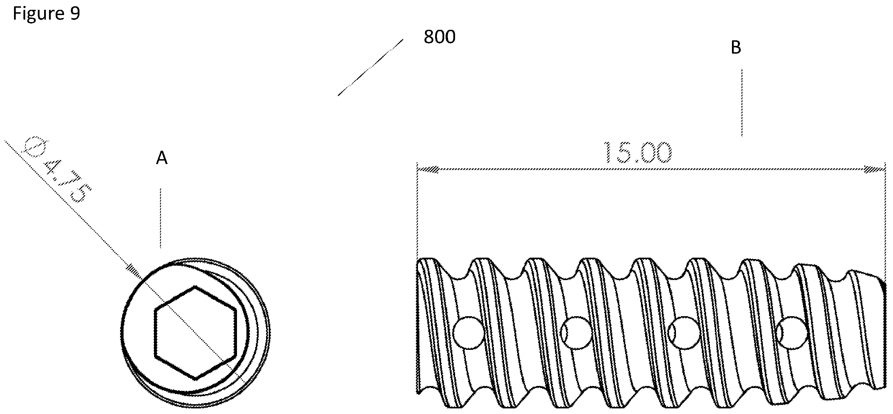

Figure 8 shows a general perspective view of the suture anchor 800. Figure 9 details the outer dimensions of the anchor 800, with an end view ("A") and a side view ("B"). Figure 9 shows exemplary dimensions; other suitable dimensions are possible and could be used in place of these dimensions. As shown, a diameter of the end of the anchor 800 is 0.475 mm, while a length of the anchor 800 is 15 mm.

Material composite comprised PLDLA 70/30 polymer reinforced with 45-50% w/w continuous mineral fibers. The mineral fibers' composition was approximately Na₂O 14%, MgO 5.4%, CaO 9%, B₂O₃ 2.3%, P₂O₅ 1.5%, and SiO₂ 67.8% w/w. The samples were manufactured by compression molding fiber bundle reinforced biocomposite material into a designated single cavity mold. Biocomposite material was comprised of the PLDLA polymer with embedded continuous fiber bundles. Orientation of fibers to each fiber bundle was approximately 0°.

The anchor was produced using fiber bundles of biocomposite material arranged in a loose helical formation wound throughout the implant body. During the compression molding process, the fiber bundles' orientation to the implant axis is amorphous (i.e. not specifically aligned).

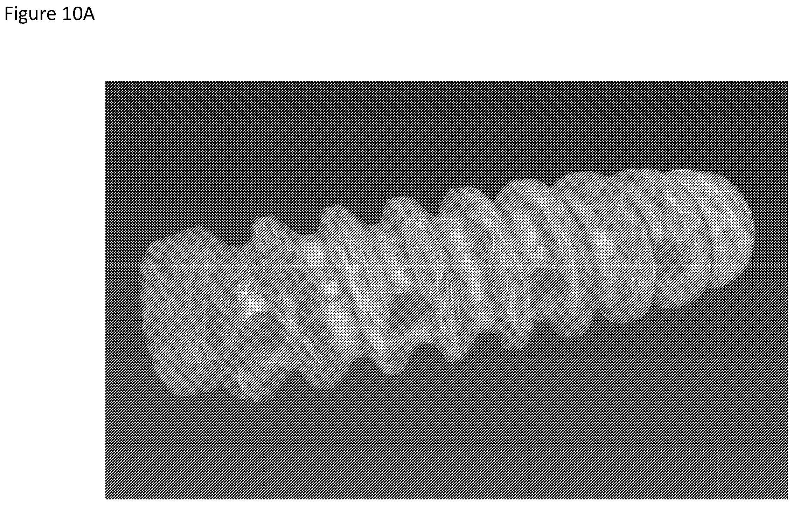

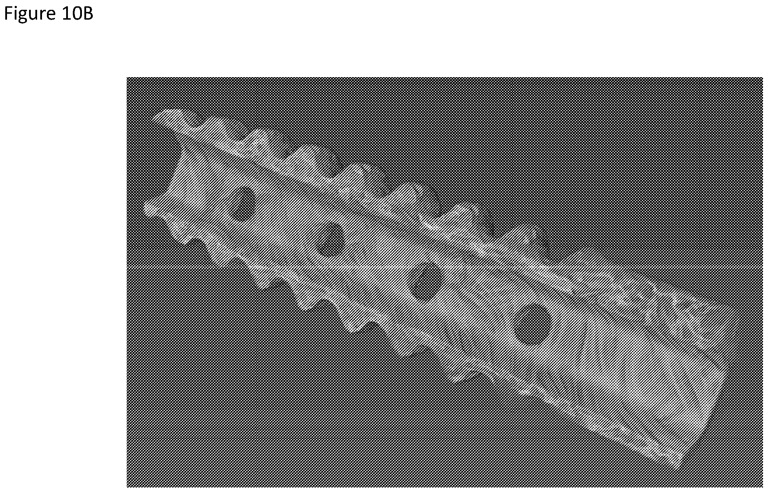

A micro-CT scan allows better understanding of the resultant fiber bundles orientation within the suture anchor implant. Figures 10A and 10B show micro-CT scans of the suture anchor implant, as a whole (Figure 10A) and in cross-section (Figure 10B). Both figures show a micro CT visualization of fiber bundles running throughout the implant. At this resolution the individual fibers within the bundles cannot be seen. Each component that appears to be a fiber is actually a fiber bundle, composed of a plurality of fibers (not visible in these images).

The micro CT shows that the general orientation of the fiber bundles inside the suture anchor is circumferential. Fibers diameter within each bundle ranges from 8µm to 18 µm. The number of fibers within fiber bundles ranges from 10 fibers to approximately 200 fibers per bundle. The distance between fibers within the bundle ranges from 0-25 µm. The distance between one fiber bundle to another ranges from 0-50 µm.

### Methods

The suture anchor screws were tested for pull-out and torque application test to failure.

The pull-out test was conducted on a 15 PCF sawbone. The suture anchor was screwed in to a 30x30x30mm sawbone block with the suture wrapped inside the anchor screw. The sawbone block was then placed on a designated jig for the test. The suture loop is then connected to the upper jig of the tensile machine. The tension is applied at a rate of 12 mm/min until failure occurred. Load and displacement were measured and recorded by the TestResources Single Column Test Machine, model 220 Frame-1505017-10F.

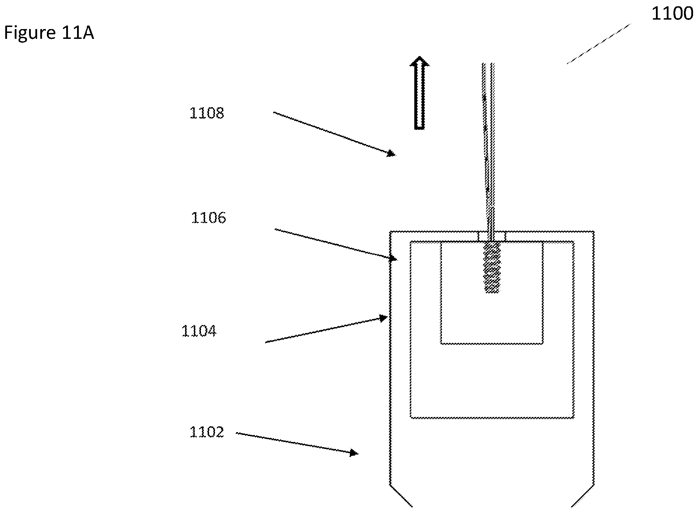

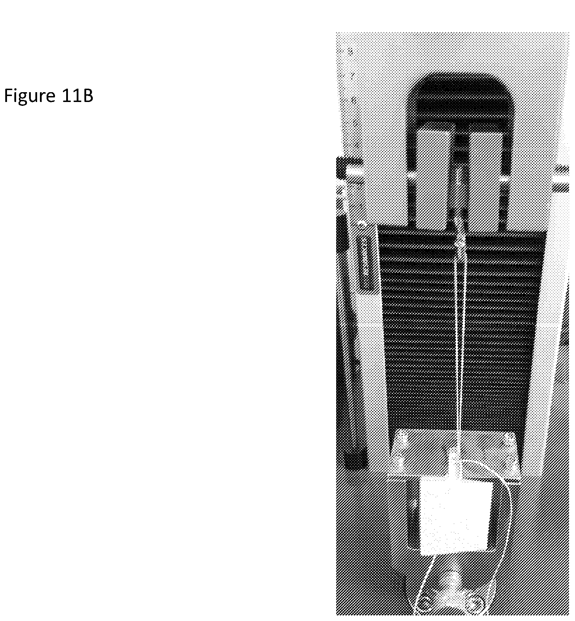

Figures 11A and 11B depict the test apparatus for the suture anchor pull-out test. Figure 11A shows a schematic of the test apparatus, while Figure 11B shows a photo of the actual device. As shown in Figure 11A, a test apparatus 1100 features a jig 1102, which holds a sawbone block 1104. A suture anchor 1106, which is the DUT (device under test), is inserted into sawbone block 1104, thereby imitating the actual use of such a suture anchor 1106. A suture loop 1108 is then attached to suture anchor 1106. Tension is applied to suture anchor 1106 in the direction of the arrow, until failure occurs, as described above.

The torque test to failure was conducted on a 30 PCF. A leading 4.2mm hole was pre-drilled in a 30x30x30mm sawbone block. Using a designated screwdriver instrument, the suture anchor screw was partially inserted into the hole. The screwdriver, with the suture anchor and the sawbone block, were then assembled on the torsion machine. The screwdriver was connected to the torsion machine rotation chuck and the sawbone block was fixed in order to prevent its rotational movement though the test. The rotation of the screwdriver was applied at a rate of 1800 deg/min, until failure occurred. Torque and angle were measured and recorded by the TestResources Torsion Test Machine, 160 Series frame, model 160 GT20 (Test Resources, Shakopee, MN, USA).

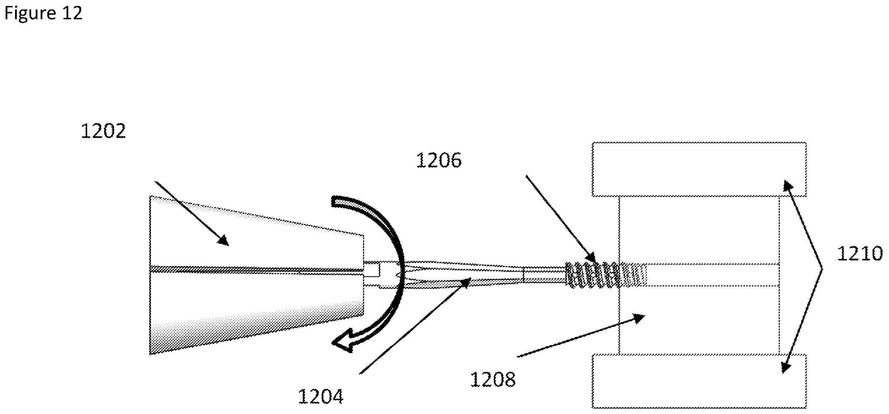

Figure 12 depicts the test apparatus for the suture anchor torsion to failure. A test apparatus 1200 features a torsion machine chuck 1202 for applying torsion as shown in the direction of the arrow. Torsion machine chuck 1202 is attached to a screwdriver 1204, which in turn is attached to a suture anchor 1206. Suture anchor 1206 is again attached to a sawbone block 1208 as previously described (the lighter color of suture anchor 1206 is to indicate that it is held within sawbone block 1208; that portion of suture anchor 1206 that is inserted would not necessarily be visible during the test). Sawbone block 1208 is attached to fixing plates 1210, to maintain immobility of sawbone block 1208 during application of torsion. Torsion was applied as described above until failure occurred.

### Results

The test results are detailed in Table 2 below.

**Table 2 shows the suture anchor test results summary, from the pull-out and torsion to failure tests.**

| Specimen type | Drive in torque [Ncm] | Maximal torque [Ncm] | Pull-out force [N] |
|---|---|---|---|
| Fiber bundle suture anchor | 30 | 56 | 118 |
| Polymeric suture anchor | 30 | 30 | 91 |

### Example #4 - Biocomposite Medical Implant Raw Material - Coated Glass Fiber

This Example relates to a single coated glass fiber, as a raw material for the production of Biocomposite medical implants.

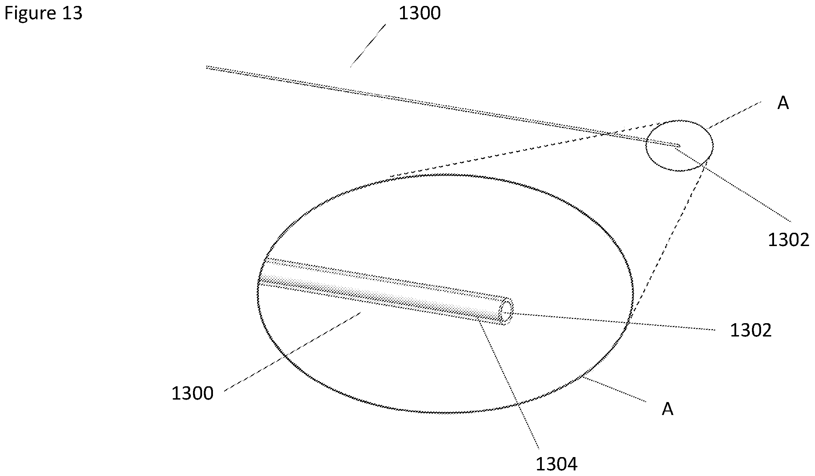

The single glass fiber is coated with a designated compatibilizer material. The outer diameter of a single glass fiber ranges from 9µm to 17µm. Figure 13 illustrates the single glass fiber geometry as a non-limiting example. Figure 13 shows a single glass fiber 1300, with an end 1302 shown in close-up in "A". A coating 1304 on glass fiber 1300 is shown.

The multiple glass fibers raw material can be grouped into a bundle. This fiber bundle is incorporated inside the biocomposite medical implant in a variety of options. Among the fiber bundle parameters which can be controlled on the raw material production process: number of fibers on each bundle, distance between fibers inside the bundle, distance between bundles, fibers alignment relative to bundle axis (straight/ low angle helix/ high angle helix), fiber bundle diameter, fiber bundle aspect ratio (major axis to minor axis ratio). The final fiber bundle type inside the medical implant can contribute to the implant performance, from both a biomechanical prospective and tissue growth prospective.

### Methods

Three single glass fibers were tested for tensile strength, each fiber separately. The average single glass fiber ultimate tensile strength (UTS) was then calculated per fiber. A total of nine (9) single fiber specimens were measured and tested. The average outer diameter for a single glass fiber was 14-16 µm. For raw material comparison, pure polymeric plates and biocomposite fiber bundle plates were tested for the same parameter.

The PLDLA plates testing samples were manufactured by compression molding of PLDLA resin into a designated single cavity mold. The PLDLA resin was weighed and placed inside the mold cavity, then heated with no active pressure. After reaching the desired temperature, pressure was applied for 5 minutes. Pressure was kept also through the cooling of the mold back to room temperature.

The biocomposite fiber bundle plates comprised PLDLA 70/30 polymer reinforced with 50% w/w continuous mineral fibers. The mineral fibers' composition was approximately Na2O 14%, MgO 5.4%, CaO 9%, B2O3 2.3%, P2O5 1.5%, and SiO₂ 67.8% w/w. The plate testing samples were manufactured by compression molding of biocomposite material containing polymer and continuous mineral fibers arranged into fiber bundles into a designated single cavity mold. Orientation of fibers to each fiber bundle was approximately 0°.

Table 3 details the averaged actual specimen dimensions per plate type, for PLDLA plates (top) and fiber bundle plates (bottom).

**Table 3**

| Plate type | Weight [g] | L [mm] | W [mm] | D [mm] |
|---|---|---|---|---|
| PLDLA | 1.010 ±0.001 | 60.11 ± 0.07 | 12.76 ± 0.02 | 1.07 ± 0.01 |
| Fiber bundle | 0.632 ± 0.081 | 51.03 ± 0.20 | 12.57 ± 0.29 | 0.67 ± 0.06 |

Four (4) samples were tested for tensile strength, tensile modulus and maximum load according to modified ASTM D3039M with a 5KN load cell and an appropriate fixture (220Q1125-95, TestResources, MN, USA). The cross head speed was set at 2 mm/min. Dimensions, weight and density of samples were recorded.

The specimens were clamped to the top and bottom clamps with a 10mm of the specimen on each clamp. The tension was applied at a rate of 2 mm/min. Load and displacement were measured and recorded by the TestResources Single Column Test Machine, model 220 Frame-1505017-10F.

### Results

Tables 4A and 4B show the mean values and standard deviations of the mechanical properties and bulk properties of the PLDLA plates (n=4), fiber bundle plates (n=4) (both Table 4A), and for a single glass fiber (n=9) (Table 4B).

**Table 4A**

| Type | Plate No. | Density [g/mm3] | Max Load [N] | Tensile strength [MPa] | Ultimate tensile strain | E [MPa] |
|---|---|---|---|---|---|---|
| PLDLA plate | 1 | 0.00126 | 826.80 | 61.76 | 0.029 | 2513.05 |
| | 2 | 0.00122 | 750.04 | 54.30 | 0.023 | 2737.26 |
| | 3 | 0.00123 | 648.26 | 47.55 | 0.016 | 3183.23 |
| | 4 | 0.00122 | 735.28 | 53.27 | 0.025 | 2958.32 |
| | Avg. | 0.00123 | 740.10 | 54.22 | 0.023 | 2847.97 |
| | STD | 0.00002 | 73.20 | 5.84 | 0.006 | 288.10 |
| Fiber bundle plate | 1 | 0.00152 | 869.76 | 91.83 | 0.072 | 6892.76 |
| | 2 | 0.00145 | 719.52 | 80.77 | 0.062 | 7648.27 |
| | 3 | 0.00146 | 775.77 | 96.81 | 0.076 | 7994.67 |
| | 4 | 0.00144 | 644.93 | 89.20 | 0.091 | 8265.68 |
| | Avg. | 0.00147 | 752.50 | 89.65 | 0.075 | 7700.35 |
| | STD | 0.00003 | 94.78 | 6.71 | 0.012 | 594.74 |

**Table 4B**

| Type | Batch | # Tested samples | Average Fiber outer diameter [µm] | Average single fiber tensile strength [MPa] |
|---|---|---|---|---|
| Single glass fiber | F0001 | 3 | 16 | 1791 |
| | F0002 | 3 | 15 | 1945 |
| | F0004 | 3 | 15 | 1771 |
| | Avg. | - | 15.33 | 1835.67 |
| | STD | - | 0.58 | 95.21 |

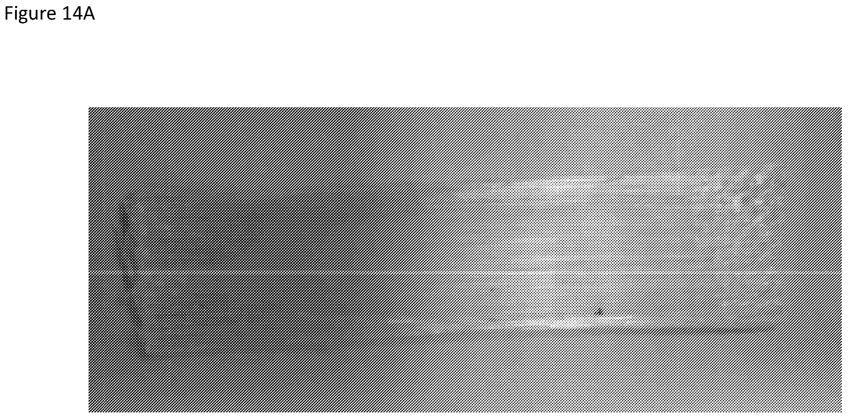

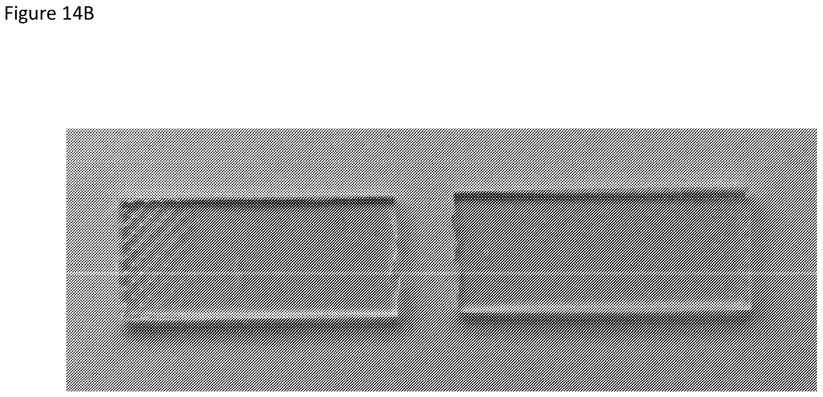

The PLDLA plate mode of failure was complete detachment of the plate as a result of the building tension. The fiber bundle plate showed no such failure, the plate kept its original form with no outer detachments or tears. Figures 14A and 14B show the mode of failure for the two plate types, specifically for the fiber bundle plate (Figure 14A) and the PLDLA plate (Figure 14B).

### SUMMARY OF RESULTS

The fiber bundle plate tensile test resulted in a tensile strength ~65% higher and young modulus ~170% higher compared to the PLDLA (layers) plate. These results highlight the contribution of the fiber bundles embedded in the plate to the mechanical properties of the plate. Thus, the fiber bundles plate had significantly greater tensile strength that the layers plate.

The mode of failure of the fiber bundle plate was also different. The PLDLA plate failed, resulting in a complete material detachment. The failure of the fiber bundle plate was not visually seen, which mean that the fiber bundles remained in their load bearing state and were not torn during the tensile test. Rather, this type of failure can be attributed to the connection between the bundles and the surrounding PLDLA polymer, a failure which can still enable the implant to provide support and tensile strength.

It will be appreciated that various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination. It will also be appreciated by persons skilled in the art that the present invention is not limited by what has been particularly shown and described hereinabove. Rather the scope of the invention is defined only by the claims which follow.

## Claims

1. A medical implant, comprising a plurality of reinforcing fiber bundles, each fiber bundle having an axis (102), comprising a plurality of fibers aligned along the axis of the bundle within 0 to 5 degrees of the axis, and a polymer binding said fiber bundles; wherein said polymer and said fiber bundles are biodegradable; wherein said fibers are separated by no more than 100 microns within each bundle;
wherein at least a portion of the reinforcing fibers are of a continuous length wherein said length is at least 100% of the length of the medical implant and is up to 10,000% of the length of the implant;
wherein said fibers comprise a reinforcing mineral composition; and wherein mineral content within the implant is in the range of 40%-60% w/w; 45%-55% w/w; 40%-70% w/w; or 50%-70% w/w;
additionally comprising a compatibilizer wherein weight content of the compatibilizer is less than 0.5% w/w; and
wherein the polymer comprises L and D isomers of poly lactic acid polymers; and/or wherein the ratio of L:D isomer of the polymer is in the range of 60:40 to 98:2; or 70:30 to 96:4.

2. The medical implant of claim 1, wherein said fiber bundles are embedded in said polymer; or said fiber bundles are mixed with said polymer.

3. The medical implant of any of the above claims, wherein said alignment of said fibers relative to the fiber bundle axis (102) is between 0 to 1 degree; and/or the distance between fibers within the bundle is in the range of 0-50 microns; 0-30 microns; 0-20 microns; or 0-10 microns.

4. The medical implant of any of the above claims wherein the fiber bundles within the medical implant are separated by less than 200 microns; 5-60 microns; 10-40 microns; 10-30 microns; or 10-50 microns.

5. The medical implant of any of the above claims wherein adjacent fiber bundles within the medical implant are off set to each other by an angle of 15 to 75 degrees; or an angle of 30 to 60 degrees.

6. The medical implant of any of the above claims, wherein the polymer comprises polylactides (PLA), poly-L-lactide (PLLA), poly-DL-lactide (PDLLA), poly-LD-lactide (PLDLA); polyglycolide (PGA); copolymers of glycolide, glycolide/trimethylene carbonate copolymers (PGA/TMC); other copolymers of PLA, such as lactide/tetramethylglycolide copolymers, lactide/trimethylene carbonate copolymers, lactide/d-valerolactone copolymers, lactide/ ε-caprolactone copolymers, L-lactide/DL-lactide copolymers, glycolide/L-lactide copolymers (PGA/PLLA), polylactideco- glycolide;
terpolymers of PLA, such as lactide/glycolide/trimethylene carbonate terpolymers, lactide/glycolide/ ε - caprolactone terpolymers, PLA/polyethylene oxide copolymers; polydepsipeptides; unsymmetrically - 3,6-substituted poly-1 ,4-dioxane-2,5-diones; polyhydroxyalkanoates; such as polyhydroxybutyrates (PHB); PHB/b-hydroxyvalerate copolymers (PHB/PHV); poly-b-hydroxypropionate (PHPA); poly-p-dioxanone (PDS); pol y-d-valerolactone - poly-ε-capralactone, poly( ε-caprolactone-D L-lactide) copolymers; methylmethacrylate-N-vinyl pyrrolidone copolymers; polyesteramides; polyesters of oxalic acid; polydihydropyrans; polyalkyl-2- cyanoacrylates; polyurethanes (PU); polyvinylalcohol (PVA); polypeptides; poly-b-malic acid (PMLA): poly-b-alkanbic acids; polycarbonates;
polyorthoesters; polyphosphates; poly(ester anhydrides); and mixtures thereof; and natural polymers, such as sugars; starch, cellulose and cellulose derivatives, polysaccharides, collagen, chitosan, fibrin, hyalyronic acid, polypeptides and proteins, or a mixture thereof

7. The medical implant of any of the above claims, wherein each fiber bundle comprises between 3500 reinforcing fibers; between 20-300 reinforcing fibers; between 25-200 reinforcing fibers; between 3-100 reinforcing fibers; between 5-50 reinforcing fibers; or between 8-16 reinforcing fibers in each bundle.

8. The medical implant of any of the above claims, wherein the diameter of the bundle is from 35 to 6500 microns; from 250 to 4000 microns; from 325 to 2600 microns; from 35 to 1300 microns; from 65 to 650 microns; or from 100 to 200 microns.

9. The medical implant of any of the above claims, wherein the fiber bundles are circular in shape; or wherein the fiber bundles are ovular in shape; optionally wherein said ovular shape comprises a 6:1; 4:1; 3:1; 2:1; or 1:1 ratio of fibers in x-axis to y-axis.

10. The medical implant of any of the above claims, wherein the fiber bundles have a geometry wherein a diameter in any axis of the bundle passing through the center is within 4 times the length; or within 2 times the length of the diameter in any other axis; wherein said diameter is identical; or wherein an average diameter of the fiber bundles is in the range of 0.5mm-10mm; 1mm-5mm; or 1.5mm-3.5mm.

11. The medical implant of any of the above claims, wherein a fiber density within each fiber bundle is in the range of 30%-99% or 40%-95% in terms of average cross-sectional area percentage or in terms of volume percentage.

12. The medical implant of any of the above claims, wherein said fibers are longer than 4 mm; 8 mm; 12 mm; 16 mm; or 20 mm.

13. The medical implant of claim 1, wherein said length is up to 1000% of the length of the implant; up to 500% of the length of the implant; up to 450% of the length of the implant; up to 400% of the length of the implant; up to 350% of the length of the implant; up to 300% of the length of the implant; up to 250% of the length of the implant; or up to 200% of the length of the implant.

14. The medical implant of any of the above claims, wherein an average diameter of reinforcing fiber is in the range of 0.1-100 µm; 1-20 µm; or 8-18 µm; wherein a standard deviation of fiber diameter between fibers within the medical implant is less than 5 µm; 3 µm; or 1.5 µm; or wherein a distance between adjacent reinforcing fibers within a biocomposite bundle is in the range of 0-50 µm; 1-30 µm; 1-20 µm; 0-25 µm; 0-15 µm; or 0-10 µm.

15. The medical implant of any of the above claims, wherein a weight percentage of reinforcing fibers within the biocomposite medical implant is in the range of 20%-90%; 40%-70%; or 40%-60%; or wherein a volume percentage of reinforcing fibers within the biocomposite medical implant is in the range of 10%-80%; or 20%-50%.

## Patentansprüche

1. Medizinisches Implantat, umfassend eine Vielzahl von Verstärkungsfaserbündeln, wobei jedes Faserbündel eine Achse (102) aufweist und eine Vielzahl von Fasern umfasst, die entlang der Achse des Bündels innerhalb von 0 bis 5 Grad zur Achse ausgerichtet sind, sowie ein Polymer, das diese Faserbündel bindet; wobei das Polymer und die Faserbündel biologisch abbaubar sind; wobei die Fasern innerhalb jedes Bündels durch nicht mehr als 100 Mikrometer voneinander getrennt sind; wobei zumindest ein Teil der Verstärkungsfasern eine kontinuierliche Länge aufweist, wobei diese Länge mindestens 100 % der Länge des medizinischen Implantats beträgt und bis zu 10.000 % der Länge des Implantats betragen kann; wobei die Fasern eine verstärkende mineralische Zusammensetzung umfassen; und wobei der Mineralgehalt innerhalb des Implantats im Bereich von 40-60 Gew.-%; 45-55 Gew.-%; 40-70 Gew.-%; oder 50-70 Gew.-% liegt; zusätzlich umfassend einen Kompatibilisator, wobei der Gewichtsanteil des Kompatibilisators weniger als 0,5 Gew.-% beträgt; und
wobei das Polymer L- und D-Isomere von Polymilchsäurepolymeren umfasst; und/oder
wobei das Verhältnis der L:D-Isomere des Polymers im Bereich von 60:40 bis 98:2 oder von 70:30 bis 96:4 liegt.

2. Medizinisches Implantat nach Anspruch 1, wobei die Faserbündel in das Polymer eingebettet sind; oder wobei die Faserbündel mit dem Polymer vermischt sind.

3. Medizinisches Implantat nach einem der vorstehenden Ansprüche, wobei die Ausrichtung der Fasern relativ zur Achse (102) des Faserbündels zwischen 0 und 1 Grad liegt; und/oder der Abstand zwischen den Fasern innerhalb des Bündels im Bereich von 0-50 Mikrometern; 0-30 Mikrometern; 0-20 Mikrometern; oder 0-10 Mikrometern liegt.

4. Medizinisches Implantat nach einem der vorstehenden Ansprüche, wobei die Faserbündel innerhalb des medizinischen Implantats durch weniger als 200 Mikrometer; 5-60 Mikrometer; 10-40 Mikrometer; 10-30 Mikrometer; oder 10-50 Mikrometer voneinander getrennt sind.

5. Medizinisches Implantat nach einem der vorstehenden Ansprüche, wobei benachbarte Faserbündel innerhalb des medizinischen Implantats gegeneinander um einen Winkel von 15 bis 75 Grad oder um einen Winkel von 30 bis 60 Grad versetzt sind.

6. Medizinisches Implantat nach einem der vorstehenden Ansprüche, wobei das Polymer Polylactide (PLA), Poly-L-lactid (PLLA), Poly-DL-lactid (PDLLA), Poly-LD-lactid (PLDLA); Polyglycolid (PGA); Copolymere von Glycolid, Glycolid/Trimethylencarbonat-Copolymere (PGA/TMC); andere Copolymere von PLA, wie Lactid/Tetramethylglycolid-Copolymere, Lactid/Trimethylencarbonat-Copolymere, Lactid/δ-Valerolacton-Copolymere, Lactid/ε-Caprolacton-Copolymere, L-Lactid/DL-Lactid-Copolymere, Glycolid/L-Lactid-Copolymere (PGA/PLLA), Polylactid-co-Glycolid; Terpolymere von PLA, wie Lactid/Glycolid/Trimethylencarbonat-Terpolymere, Lactid/Glycolid/ε-Caprolacton-Terpolymere, PLA/Polyethylenoxid-Copolymere; Polydepsipeptide; unsymmetrisch 3,6-substituierte Poly-1,4-dioxan-2,5-dione; Polyhydroxyalkanoate; wie Polyhydroxybutyrate **(PHB);** PHB/β-Hydroxyvalerat-Copolymere **(PHB/PHV);** Poly-β-Hydroxypropionat (**PHPA**); Poly-p-Dioxanon **(PDS);** Poly-δ-Valerolacton- Poly-ε-Caprolacton, Poly(ε-Caprolacton-DL-Lactid)-Copolymere; Methylmethacrylat-N-vinylpyrrolidon-Copolymere; Polyesteramide; Polyester der Oxalsäure; Polydihydropyrane; Polyalkyl-2-cyanoacrylate; Polyurethane (PU); Polyvinylalkohol (PVA); Polypeptide; Poly-β-Äpfelsäure (PMLA); Poly-β-alkansäuren; Polycarbonate; Polyorthoester; Polyphosphate; Poly(esteranhydride); und Mischungen davon; sowie natürliche Polymere, wie Zucker; Stärke, Cellulose und Cellulosederivate, Polysaccharide, Kollagen, Chitosan, Fibrin, Hyaluronsäure, Polypeptide und Proteine oder eine Mischung davon.

7. Medizinisches Implantat nach einem der vorstehenden Ansprüche, wobei jedes Faserbündel zwischen 3 und 500 Verstärkungsfasern; zwischen 20 und 300 Verstärkungsfasern; zwischen 25 und 200 Verstärkungsfasern; zwischen 3 und 100 Verstärkungsfasern; zwischen 5 und 50 Verstärkungsfasern; oder zwischen 8 und 16 Verstärkungsfasern in jedem Bündel umfasst.

8. Medizinisches Implantat nach einem der vorstehenden Ansprüche, wobei der Durchmesser des Bündels 35 bis 6500 Mikrometer; 250 bis 4000 Mikrometer; 325 bis 2600 Mikrometer; 35 bis 1300 Mikrometer; 65 bis 650 Mikrometer; oder 100 bis 200 Mikrometer beträgt.

9. Medizinisches Implantat nach einem der vorstehenden Ansprüche, wobei die Faserbündel kreisförmig sind; oder wobei die Faserbündel oval geformt sind; optional wobei die ovale Form ein Verhältnis der Fasern in der x-Achse zur y-Achse von 6:1; 4:1; 3:1; 2:1; oder 1:1 umfasst.

10. Medizinisches Implantat nach einem der vorstehenden Ansprüche, wobei die Faserbündel eine Geometrie aufweisen, bei der ein Durchmesser einer beliebigen Achse des Bündels, die durch das Zentrum verläuft, innerhalb des 4-Fachen der Länge oder innerhalb des 2-Fachen der Länge des Durchmessers in einer beliebigen anderen Achse liegt; wobei dieser Durchmesser identisch ist; oder wobei ein mittlerer Durchmesser der Faserbündel im Bereich von 0,5 mm-10 mm; 1 mm-5 mm; oder 1,5 mm-3,5 mm liegt.

11. Medizinisches Implantat nach einem der vorstehenden Ansprüche, wobei eine Faserdichte innerhalb jedes Faserbündels im Bereich von 30-99 % oder 40-95 % liegt, ausgedrückt als prozentualer Anteil der mittleren Querschnittsfläche oder als Volumenanteil.

12. Medizinisches Implantat nach einem der vorstehenden Ansprüche, wobei die Fasern länger als 4 mm; 8 mm; 12 mm; 16 mm; oder 20 mm sind.

13. Medizinisches Implantat nach Anspruch 1, wobei diese Länge bis zu 1000 % der Länge des Implantats; bis zu 500 % der Länge des Implantats; bis zu 450 % der Länge des Implantats; bis zu 400 % der Länge des Implantats; bis zu 350 % der Länge des Implantats; bis zu 300 % der Länge des Implantats; bis zu 250 % der Länge des Implantats; oder bis zu 200 % der Länge des Implantats beträgt.

14. Medizinisches Implantat nach einem der vorstehenden Ansprüche, wobei ein mittlerer Durchmesser der Verstärkungsfasern im Bereich von 0,1-100 µm; 1-20 µm; oder 8-18 µm liegt; wobei eine Standardabweichung des Faserdurchmessers zwischen den Fasern innerhalb des medizinischen Implantats weniger als 5 µm; 3 µm; oder 1,5 µm beträgt; oder wobei ein Abstand zwischen benachbarten Verstärkungsfasern innerhalb eines Biokomposit-Bündels im Bereich von 0-50 µm; 1-30 µm; 1-20 µm; 0-25 µm; 0-15 µm; oder 0-10 µm liegt.

15. Medizinisches Implantat nach einem der vorstehenden Ansprüche, wobei ein Gewichtsanteil der Verstärkungsfasern innerhalb des biokompositen medizinischen Implantats im Bereich von 20-90 %; 40-70 %; oder 40-60 % liegt; oder wobei ein Volumenanteil der Verstärkungsfasern innerhalb des biokompositen medizinischen Implantats im Bereich von 10-80 % oder 20-50 % liegt.

## Revendications

1. Implant médical, comprenant une pluralité de faisceaux de fibres de renforcement, chaque faisceau de fibres ayant un axe (102), comprenant une pluralité de fibres alignées le long de l'axe du faisceau à moins de 5 degrés de l'axe, et un polymère liant lesdits faisceaux de fibres ; dans lequel ledit polymère et lesdits faisceaux de fibres sont biodégradables ; dans lequel lesdites fibres sont séparées par moins de 100 microns dans chaque faisceau ; dans lequel au moins une partie des fibres de renfort sont d'une longueur continue, ladite longueur étant d'au moins 100 % de la longueur de l'implant médical et pouvant atteindre 10 000 % de la longueur de l'implant ; dans lequel lesdites fibres comprennent une composition minérale de renfort ; et dans lequel la teneur en minéraux dans l'implant est comprise entre 40 % et 60 % masse/masse, 45 % et 55 % masse/masse, 40 % à 70 % masse/masse ou 50 % à 70 % masse/masse, comprenant en outre un agent de compatibilité dont la teneur en poids est inférieure à 0,5 % masse/masse; et
dans lequel le polymère comprend des isomères L et D de polymères d'acide polylactique; et/ou dans lequel le rapport des isomères L:D du polymère est compris entre 60:40 et 98:2 ou entre 70:30 et 96:4.

2. Implant médical selon l'indication 1, dans lequel lesdits faisceaux de fibres sont noyés dans ledit polymère ; ou lesdits faisceaux de fibres sont mélangés avec ledit polymère.

3. Implant médical selon l'une des indications précédentes, dans lequel ledit alignement desdites fibres par rapport à l'axe du faisceau de fibres (102) est compris entre 0 et 1 degré ; et/ou la distance entre les fibres au sein du faisceau est comprise dans la plage de 0 à 50 microns, 0 à 30 microns, 0 à 20 microns ou 0 à 10 microns.

4. Implant médical selon l'une des indications précédentes, dans lequel les faisceaux de fibres à l'intérieur de l'implant médical sont à une distance de moins de 200 microns, 5 à 60 microns, 10 à 40 microns, 10 à 30 microns ou 10 à 50 microns.

5. Implant médical selon l'une des indications précédentes, dans lequel les faisceaux de fibres adjacents à l'intérieur de l'implant médical sont décalés les uns par rapport aux autres d'un angle de 15 à 75 degrés ; ou d'un angle de 30 à 60 degrés.

6. Implant médical selon l'une des indications précédentes, dans lequel le polymère comprend des polylactides (PLA), du poly-L-lactide (PLLA), du poly-DL-lactide (PDLLA), du poly-LD-lactide (PLDLA) ; du polyglycolide (PGA) ; des copolymères de glycolide, des copolymères de glycolide/carbonate de triméthylène (PGA/TMC) ; d'autres copolymères de PLA, tels que des copolymères de lactide/tétraméthylglycolide, des copolymères de lactide/carbonate de triméthylène, des copolymères de lactide/d-valérolactone, des copolymères de lactide/ε-caprolactone, copolymères de L-lactide/DL-lactide, copolymères de glycolide/L-lactide (PGA/PLLA), polylactide-coglycolide ; terpolymères de PLA, tels que les terpolymères de lactide/glycolide/carbonate de triméthylène, les terpolymères de lactide/glycolide/ε -caprolactone, les copolymères de PLA/polyéthylèneoxyde ; polydepsipeptides ; poly-I ,4-dioxane-2,5-diones asymétriquement substituées en position 3,6 ; polyhydroxyalcanoates ; tels que les polyhydroxybutyrates (PHB) ; copolymères PHB/b-hydroxyvalérate (PHB/PHV) ; poly-b-hydroxypropionate (PHPA) ; poly-p-dioxanone (PDS) ; poly-d-valérolactone - poly-ε-capralactone, copolymères poly(ε-caprolactone-D L-lactide) ; copolymères méthacrylate de méthyle-N-vinylpyrrolidone ; polyesteramides ; polyesters d'acide oxalique ; polydihydropyranes ; polyalkyl-2-cyanoacrylates ; polyuréthanes (PU) ; alcool polyvinylique (PVA) ; polypeptides ; poly-b-acide malique (PMLA) ; poly-b-acides alcaniques ; polycarbonates ; polyorthoesters ; polyphosphates ; poly(anhydrides d'esters) ; et leurs mélanges ; et polymères naturels, tels que les sucres ; l'amidon, la cellulose et les dérivés de cellulose, les polysaccharides, le collagène, le chitosane, la fibrine, l'acide hyaluronique, les polypeptides et les protéines, ou un mélange de ceux-ci.

7. Implant médical selon l'une des indications précédentes, dans lequel chaque faisceau de fibres comprend entre 3500 fibres de renfort,entre 20 et 300 fibres de renfort, entre 25 et 200 fibres de renfort, entre 3 et 100 fibres de renfort, entre 5 et 50 fibres de renfort ou entre 8 et 16 fibres de renfort dans chaque faisceau.

8. Implant médical selon l'une des indications précédentes, dans lequel le diamètre du faisceau est compris entre 35 et 6500 microns, entre 250 et 4000 microns, entre 325 et 2600 microns, entre 35 et 1300 microns, entre 65 et 650 microns ou entre 100 et 200 microns.

9. Implant médical selon l'une des indications précédentes, dans lequel les faisceaux de fibres sont de forme circulaire ; ou dans lequel les faisceaux de fibres sont de forme ovale ; éventuellement dans lequel ladite forme ovale comprend un rapport de 6:1, 4:1, 3:1, 2:1 ou 1:1 entre les fibres dans l'axe x et celles dans l'axe y.

10. Implant médical selon l'une des indications précédentes, dans lequel les faisceaux de fibres ont une géométrie dans laquelle un diamètre dans n'importe quel axe du faisceau passant par le centre est compris dans un rapport de 4 fois la longueur ; ou dans un rapport de 2 fois la longueur du diamètre dans n'importe quel autre axe ; dans lequel ledit diamètre est identique ; ou dans lequel un diamètre moyen des faisceaux de fibres est compris dans la plage de 0,5 à 10 mm ; de 1 à 5 mm ; ou de 1,5 à 3,5 mm.

11. Implant médical selon l'une des indications précédentes, dans lequel la densité des fibres dans chaque faisceau de fibres est comprise entre 30 % et 99 % ou entre 40 % et 95 % en termes de pourcentage de section transversale moyenne ou en termes de pourcentage de volume.

12. Implant médical selon l'une des indications précédentes, dans lequel lesdites fibres ont une longueur supérieure à 4 mm, 8 mm, 12 mm, 16 mm ou 20 mm.

13. Implant médical selon l'indication 1, dans lequel ladite longueur est jusqu'à 1000 % de la longueur de l'implant ; jusqu'à 500 % de la longueur de l'implant ; jusqu'à 450 % de la longueur de l'implant ; jusqu'à 400 % de la longueur de l'implant ; jusqu'à 350 % de la longueur de l'implant ; jusqu'à 300 % de la longueur de l'implant ; jusqu'à 250 % de la longueur de l'implant ; ou jusqu'à 200 % de la longueur de l'implant.

14. Implant médical selon l'une des indications ci-dessus, dans lequel le diamètre moyen de la fibre de renforcement est compris dans la plage de 0,1 à 100 µm, 1 à 20 µm ou 8 à 18 µm ; dans lequel l'écart type du diamètre des fibres entre les fibres à l'intérieur de l'implant médical est inférieur à 5 µm, 3 µm ou 1,5 µm ; ou dans lequel la distance entre les fibres de renfort adjacentes à l'intérieur d'un faisceau biocomposite est comprise entre 0 et 50 µm, 1 et 30 µm, 1 et 20 µm, 0 et 25 µm, 0 et15 µm ou 0 et 10 µm.

15. Implant médical selon l'une des indications ci-dessus, dans lequel le pourcentage en poids des fibres de renfort dans l'implant médical biocomposite est compris entre 20 % et 90 %, entre 40 % et 70 % ou entre 40 % et 60 % ; ou dans lequel le pourcentage en volume des fibres de renfort dans l'implant médical biocomposite est compris entre 10 % et 80 % ou entre 20 % et 50 %.
